(19) **Europäisches Patentamt**

**European Patent Office**

**Office européen des brevets**

(11) **EP 1 506 381 B1**

(12) **EUROPÄISCHE PATENTSCHRIFT**

(45) Veröffentlichungstag und Bekanntmachung des Hinweises auf die Patenterteilung:
**12.07.2006 Patentblatt 2006/28**

(51) Int Cl.:
*G01G 17/06* (2006.01) *G01N 35/10* (2006.01)

(21) Anmeldenummer: **03718587.3**

(22) Anmeldetag: **13.05.2003**

(86) Internationale Anmeldenummer:
**PCT/CH2003/000305**

(87) Internationale Veröffentlichungsnummer:
**WO 2003/098170 (27.11.2003 Gazette 2003/48)**

(54) **VORRICHTUNG ZUR DOSIERUNG VON SUBSTANZEN**

DEVICE FOR DOSING SUBSTANCES

DISPOSITIF DE DOSAGE DE MATIERES

(84) Benannte Vertragsstaaten:
**AT BE BG CH CY CZ DE DK EE ES FI FR GB GR HU IE IT LI LU MC NL PT RO SE SI SK TR**

(30) Priorität: **17.05.2002 CH 846022002**

(43) Veröffentlichungstag der Anmeldung:
**16.02.2005 Patentblatt 2005/07**

(73) Patentinhaber: **Chemspeed Technologies AG 4302 Augst (CH)**

(72) Erfinder:
• **GUELLER, Rolf CH-5027 Herznach (CH)**

• **SCHNEIDER, Michael CH-5070 Frick (CH)**
• **SCHRÖER, Josef CH-4132 Muttenz (CH)**
• **MOOR, Christoph CH-5024 Küttigen (CH)**

(74) Vertreter: **Bollhalder, Renato et al A. Braun Braun Héritier Eschmann AG, Holbeinstrasse 36-38 4051 Basel (CH)**

(56) Entgegenhaltungen:
**EP-A- 0 616 276 EP-A- 0 731 344**
**WO-A-02/04900 WO-A-02/29369**

EP 1 506 381 B1

**Beschreibung**

Die vorliegende Erfindung bezieht sich auf eine Vorrichtung und ein Verfahren zur Dosierung von Substanzen, wie sie in den Oberbegriffen der unabhängigen Patentansprüche 1 und 26 definiert sind.

Im Labor erfolgt das Dosieren von Substanzen in Behälter in vielen Fällen durch manuelle Zugaben mit einem Spatel in den zu befüllenden Behälter oder in einen Zwischenbehälter, der anschliessend in den zu befüllenden Behälter entleert wird, wobei der Behälter oder Zwischenbehälter auf eine Waage gestellt wird. Die erreichbare Präzision ist dabei durch die Geschicklichkeit des Experimentators limitiert. Das Vorgehen ist nur mit grossem technischen Aufwand automatisierbar. Zudem lassen sich die häufig vorkommenden Überdosierungen manuell nur umständlich und automatisiert nur mit noch grösserem Aufwand korrigieren. Im Weiteren kann nicht jeder Behälter auf eine Waage gestellt werden, und selbst wenn dies möglich ist, kann nicht ortsunabhängig dosiert werden.

Aus diesen Gründen wurde von der Firma Chemspeed Ltd., CH-4302 Augst, eine in der WO 02/29369 A1 offenbarte Dosiervorrichtung entwickelt, die eine kontinuierliche, von oben gravimetrisch kontrollierte Dosierung in einen beliebigen Behälter an einem beliebigen Ort innerhalb des Arbeitsbereiches eines Roboterarmes ermöglicht. Dabei muss aber die Dosiervorrichtung auf die zu dosierende Substanz abgestimmt werden und/oder die Substanz muss bestimmte Anforderungen betreffend Rieselfähigkeit oder das Fliessverhalten erfüllen, um kontinuierlich dosiert werden zu können.

Die Schrift EP 0 731 344 A offenbart die Dosierung einer Substanz mittels einer Vielzahl einzeln entleerbarer Substanzkompartimente, wobei diese sich aus einem Haupt-Substanzkompartiment, mehreren additiven Substanzkompartimenten und mehreren subtraktiven Substanzkompartimenten zusammensetzen. Die Substanzkompartimente sind so dimensioniert, dass bei gefüllten Substanzkompartimenten die Substanz im Haupt-Substanzkompartiment und den subtraktiven Substanzkompartimenten zusammen ungefähr das Zielgewicht aufweist. Mittels einer Wägezelle werden das Haupt-Substanzkompartiment und die subtraktiven Substanzkompartimenten mit der enthaltenen Substanz einmal gewogen. Zieht man das Gewicht des Haupt-Substanzkompartiments und der subtraktiven Substanzkompartimente ab, erhält man das Rohgewicht der Substanz im Haupt-Substanzkompartiment und den subtraktiven Substanzkompartimenten. Dieses Rohgewicht wird durch Differenzbildung mit dem Zielgewicht der Substanz, d.h. der gewünschten Substanzmenge verglichen. Abhängig von der ermittelten Differenz wird dann bestimmt, welche Substanzkompartimente zusammen mit dem Haupt-Substanzkompartiment in einen Zielbehälter entleert zu werden brauchen, damit die in den Zielbehälter entleerte (dosierte) Substanz das Zielgewicht aufweist.

Bei den in EP 0 731 344 A offenbarten Dosierverfahren erfolgt die Entleerung aller bestimmter Substanzkompartimente in den Zielbehälter gleichzeitig. Danach wird die dosierte Substanz nicht mehr gewogen. Es findet keine Annäherung an das Zielgewicht durch Dosieren einer ersten Substanzmenge, Wägen der dosierten Substanz, Nachdosieren etc, statt.

Angesichts der Nachteile der bisher bekannten, oben beschriebenen Vorrichtungen und Verfahren liegt der Erfindung die folgende Aufgabe zugrunde. Zu schaffen sind eine Vorrichtung und ein Verfahren zur Dosierung von Substanzen der eingangs erwähnten Art, die für bezüglich Konsistenz, Reaktivität, Morphologie etc. unterschiedlichste Substanzen ein genaues, einfaches und automatisierbares Dosieren einer gewünschten Substanzmenge ermöglichen.

Diese Aufgabe wird durch die erfindungsgemässe Vorrichtung und das erfindungsgemässe Verfahren gelöst, wie sie in den unabhängigen Patentansprüchen 1 und 26 definiert sind. Bevorzugte Ausführungsvarianten ergeben sich aus den abhängigen Patentansprüchen.

Das Wesen der Erfindung besteht im Folgenden: Eine Vorrichtung zur Dosierung von Substanzen weist eine Substanzaufnahmeeinrichtung auf, die eine Vielzahl von Substanzkompartimenten zur Aufnahme von zu dosierender Substanz umfasst, die einzeln entleerbar sind. Die Vorrichtung umfasst ausserdem eine Entleereinrichtung zur Entleerung der Substanzkompartimente, eine Waage zur Bestimmung der Menge an dosierter Substanz und Steuermittel, die die Entleerung der Substanzkompartimente in Abhängigkeit von der mittels der Waage bestimmten Menge an dosierter Substanz steuern.

Dadurch, dass die Substanzaufnahmeeinrichtung eine Vielzahl von Substanzkompartimenten aufweist, die einzeln entleerbar sind, und eine Waage zur Bestimmung der Menge an dosierter Substanz verwendet wird, können unterschiedlichste Substanzen mittels eines Annäherungsverfahrens mit hoher Geschwindigkeit schrittweise genau dosiert werden. Die Substanzen sind beispielsweise in flüssiger, pulverförmiger oder fester Form oder sie können auch eine beliebige Mischung von festen oder flüssigen Substanzen und von unterschiedlichster Konsistenz sein. Das Annäherungsverfahren kann problemlos automatisiert werden. Ausserdem kann sehr einfach aus vielen Vorratsbehältern in viele Zielgefässe dosiert werden. Ausserdem ist die Dosierung zumindest dann nicht ortsgebunden, wenn eine Waage eingesetzt wird, die von oben das Gewicht der Substanzaufnahmeeinrichtung, der Entleereinrichtung und der in den Substanzkompartimenten vorhandenen Substanz misst.

Die Steuermittel umfassen beispielsweise eine Rechnereinheit mit einem Prozessor und elektrische Leitungen zur Waage und zur Entleereinrichtung.

Bei einer vorteilhaften Ausführungsvariante umfasst die Substanzaufnahmeeinrichtung Substanzkompartimente verschiedener Grössenklassen, mit denen verschiedene Mengen an zu dosierender Substanz aufnehmbar sind. Dies ermöglicht eine raschere Annäherung an die gewünschte Dosiermenge, da zunächst mit grösseren Substanzkompartimenten eine Grobannäherung durchführbar ist, die dann durch Entleerung kleinerer Substanzkompartimente verfeinert werden kann. Durch die verschiedenen Grössenklässen kann zudem ein grosser Bereich abgedeckt werden und dennoch mit hoher Auflösung dosiert werden.

Vorzugsweise sind zumindest einige der Grössenklassen über mindestens einen Faktor 5 abgestuft; beispielsweise im Verhältnis 1:2:5. Bei einer vorteilhaften Alternative sind die Grössenklassen über einen Faktor 9 abgestuft, beispielsweise im Verhältnis 1:3:9. Da die für die Dosierung notwendigen Berechnungen normalerweise durch einen Prozessor vorgenommen werden, sind auch beliebige nicht-ganzzahlige Verhältnisse verwendbar, die sich beispielsweise durch die Herstellung der Substanzkompartimente ergeben können.

Bei einer vorteilhaften Ausführungsvariante sind mindestens einige der Substanzkompartimente mit zu dosierender Substanz vorgefüllt und vorzugsweise verschlossen. Die Substanzkompartimente können beispielsweise vorgefüllt gekauft und/oder gelagert werden und dann bei Bedarf von der erfindungsgemässen Vorrichtung aufgenommen und entleert werden. Der Verschluss kann zum Beispiel aus einer Folie bestehen, die unmittelbar vor der Verwendung als ganzes abgezogen wird oder die alternativ dazu durch einen zum Entleeren des Substanzkompartiments eingesetzten Druckstoss oder einen anderen physikalischen oder chemischen Prozess derart geöffnet wird, dass sie vorteilhafterweise so reisst, dass keine Rückstände der Folie in die zu befüllenden Gefässe fallen. Z.B. auf einer Trägerplatte ist es auch möglich, verschiedene Substanzkompartimente mit verschiedenen Substanzen vorzubefüllen, wobei diese verschiedene physikalische und chemische Eigenschaften haben können.

Mit Vorteil sind die Substanzkompartimente durch vertikal angeordnete Röhrchen gebildet. Diese Röhrchen sind beispielsweise aus Glas, Kunststoff oder Metall, zylindrisch und werden zum Aufnehmen von Substanz vorzugsweise in die Substanz eingetaucht oder eingesteckt und wieder herausgezogen. Dies kann auf einfache Weise automatisiert erfolgen, braucht es doch hierzu nur eine Einrichtung zur vertikalen Verstellung der Substanzkompartimente oder des die Substanz enthaltenden Vorratsbehälters.

Bevorzugt weisen die Röhrchen grössenklassenweise verschiedene Innendurchmesser auf. Beim Eintauchen oder Einstechen der Röhrchen in die Substanz und anschliessendem Herausziehen der Röhrchen bleiben dann bei genügend kleinen Innendurchmessern unterschiedliche Substanzmengen in den Röhrchen verschiedener Grössenklassen hängen.

Vorteilhafterweise sind die Innendurchmesser der Röhrchen kleiner als 5 mm, vorzugsweise kleiner als 1 mm, bevorzugter kleiner als 0,5 mm, noch bevorzugter kleiner als 0,1 mm. Dadurch ist gewährleistet, dass auch sehr feine pulverförmige Substanzen sowie flüssige Substanzen von den Röhrchen aufgenommen werden können.

Bevorzugt verjüngen sich mindestens einige der Röhrchen von oben nach unten. Dadurch steht oben mehr Platz für die Entleereinrichtung oder die Aufnahme von Substanz zur Verfügung.

Bei einer vorteilhaften Ausführungsvariante sind mindestens einige der Röhrchen unten zugespitzt oder scharfkantig ausgebildet. Dies ermöglicht ein einfacheres Einstechen in pulverförmige oder feste Substanzen und führt bei flüssigen Substanzen zu einem regelmässigeren Loslösen von Tropfen, d.h. zu einem gleichmässigeren Füllstand von Röhrchen der gleichen Grössenklasse.

Bei einer vorteilhaften Ausführungsvariante sind mindestens einige der Röhrchen mit zu dosierender Substanz vorgefüllt, wobei vorzugsweise die beiden Enden der Röhrchen mit einer Folie verschlossen sind. Die Röhrchen können beispielsweise vorgefüllt gekauft und/oder gelagert werden und dann bei Bedarf von der erfindungsgemässen Vorrichtung aufgenommen und entleert werden.

Mit Vorteil weisen mindestens einige der Substanzkompartimente eine Innenfläche mit einem arithmetischen Mittenrauhwert $R_a$ grösser als 0,5 $\mu$m auf. Dadurch wird die aufgenommene Substanz gut gehalten.

Bei einer vorteilhaften Ausführungsvariante weist die erfindungsgemässe Vorrichtung verschiedene Klassen von Substanzkompartimenten mit Innenflächen mit unterschiedlichen arithmetischen Mittenrauhwerten $R_a$ auf. Da die Innenflächen der Substanzkompartimente verschiedener Klassen unterschiedliche arithmetische Mittenrauhwerte $R_a$ aufweisen, halten die betreffenden Substanzkompartimente klassenweise unterschiedliche Mengen an Substanz zurück. Dies ermöglicht eine raschere Annäherung an die gewünschte Dosiermenge.

Vorzugsweise weisen mindestens einige der Substanzkompartimente an ihrer Innenfläche flexible Lamellen und/oder Widerhaken auf. Dadurch wird die zu dosierende Substanz besser im Substanzkompartiment gehalten.

Bei einer vorteilhaften Ausführungsvariante weist die erfindungsgemässe Vorrichtung verschiedene Klassen von Substanzkompartimenten mit Innenflächen mit unterschiedlicher Benetzbarkeit auf. Dadurch können Kapillarkräfte optimal ausgenützt werden und die betreffenden Substanzkompartimente können klassenweise unterschiedliche Mengen an flüssiger Substanz zurückhalten, was eine raschere Annäherung an die gewünschte Dosiermenge ermöglicht.

Mit Vorteil ist die Substanzaufnahmeeinrichtung von der Entleereinrichtung automatisch lösbar, beispielsweise durch Abstreifen an einem fixen Teil. Substanzaufnahmeeinrichtungen können so automatisch ausgetauscht werden, beispielsweise bei einem Substanzwechsel, und allenfalls auch kostengünstig für den Einmalgebrauch ausgebildet werden, wodurch die Gefahr von Verunreinigungen durch andere zu dosierende Substanzen ausgeschlossen werden kann.

Vorzugsweise sind die Substanzkompartimente in der Substanzaufnahmeeinrichtung einzeln montiert und ist ihre Anzahl varierbar. Es können dann jeweils so viele Substanzkompartimente einer Klasse montiert werden, wie gerade benötigt werden.

Bevorzugt sind die Substanzkompartimente in der Substanzaufnahmeeinrichtung einzeln zwischen einer Füllposition, in der sie befüllbar sind, und einer inaktiven Position, in der sie nicht befüllbar sind, verstellbar montiert. Es können dann jeweils so viele Substanzkompartimente einer Klasse in die Füllposition gebracht werden, wie gerade benötigt werden. Die Verstellbarkeit der Substanzkompartimente kann beispielsweise durch ihre Lagerung in Rohren, aus denen sie ausgefahren werden können, sichergestellt werden.

Vorteilhafterweise umfasst die erfindungsgemässe Vorrichtung Mittel zur vertikalen Verstellung der Substanzaufnahmeeinrichtung. Dies ermöglicht auf einfache Weise das Eintauchen oder Einstechen der Substanzkompartimente in aufzunehmende Substanz und das wieder Herausziehen der Substanzkompartimente.

Bei einer bevorzugten Ausführungsvariante umfasst die Entleereinrichtung Mittel zur Beaufschlagung jedes einzelnen Substanzkompartiments mit Druckgas. Ein pneumatischer oder ein anderer Druckstoss kann beispielsweise durch Öffnen eines Ventils, irreversibles Zerstören eines dafür vorgesehenen Bauteils oder Entleeren eines Druckbehälters erzeugt werden. Durch die Beaufschlagung mit Druckgas kann ein Substanzkompartiment auf einfache Weise entleert werden.

Bei einer alternativen vorteilhaften Ausführungsvariante weist die Entleereinrichtung für jedes Substanzkompartiment einen verstellbaren Kolben auf. Die Substanz kann dann mit diesem Kolben aus dem Substanzkompartiment ausgestossen werden. Anstelle eines Kolbens kann auch ein anderes mechanisches Bauteil verwendet werden. Die Verstellung des Kolbens bzw. anderen mechanischen Bauteils kann beispielsweise durch Motoren, Federn, Magnete oder Piezoelemente bewirkt werden.

Bei einer weiteren alternativen Ausführungsvariante weist die Entleereinrichtung Mittel zur Veränderung der Geometrie jedes einzelnen Substanzkompartiments auf, die vorzugsweise Mittel zur Erzeugung eines mechanischen Drucks, einer elektrischen Spannung oder einer Temperaturänderung umfassen. Die Mittel zur Erzeugung eines mechanischen Drucks umfassen beispielsweise Piezoelemente, insbesondere piezokeramische Verbundelemente. Durch die Geometrieänderung kann die aufgenommene Substanz vom Substanzkompartiment gelöst und dieses so entleert werden.

Bei noch einer alternativen Ausführungsvariante weist die Entleereinrichtung Mittel zur Veränderung der Oberflächeneigenschaften der Innenfläche jedes einzelnen Substanzkompartiments auf, die vorzugsweise Mittel zur Erzeugung einer elektrischen Spannung und/oder einer Temperaturänderung umfassen. Durch die Veränderung der Oberflächeneigenschaften kann die aufgenommene Substanz vom Substanzkompartiment gelöst und dieses so entleert werden.

Bei einer weiteren alternativen Ausführungsvariante weist die Entleereinrichtung Mittel zur Veränderung der Fliesseigenschaften der zu dosierenden Substanz in jedem einzelnen Substanzkompartiment auf, die vorzugsweise Mittel zur Erzeugung einer elektrischen Spannung oder einer Temperaturänderung umfassen. Durch die Veränderung der Fliesseigenschaften der zu dosierenden Substanz kann die aufgenommene Substanz vom Substanzkompartiment gelöst und dieses so entleert werden.

Vorteilhafterweise sind die Entleereinrichtung und die Substanzaufnahmeeinrichtung an der Waage angeordnet, so dass sie von dieser gewogen werden. Die Anordnung der Entleereinrichtung und der Substanzaufnahmeeinrichtung erfolgt beispielsweise so wie dies in der WO 02/29369 A1 für eine herkömmliche Dosiervorrichtung beschrieben ist. Durch dieses Wägen von oben kann bestimmt werden, wieviel Substanz bei der Entleerung eines Substanzkompartiments abgegeben wird. Die erfindungsgemässe Vorrichtung ist so ortsunabhängiger als beim Vorhandensein einer Waage unterhalb des mit Substanz zu befüllenden Gefässes. Es kann zum Beispiel im ganzen Arbeitsbereich eines Roboterarms dosiert werden.

Alternativ zum Wägen von oben kann auch nur die Dosiervorrichtung oberhalb des zu befüllenden Gefässes angeordnet sein, wenn dieses auf einer Waage steht.

Es ist auch möglich, mit zwei Waagen zu arbeiten: an einer wird das Dosiersystem angebracht, eine zweite steht unter dem zu befüllenden Gefäss zur Kontrolle der oberen Waage.

Alternativ ist die Waage bzw. eine zweite Waage ausgebildet, um ein zu befüllendes Gefäss aufzunehmen und das Gewicht des Gefässes und der in das Gefäss dosierten Substanz zu messen. Ein Vorteil dieser Alternative ist, dass das Gewicht der bereits dosierten Substanz und nicht nur das Gewicht der abgegebenen Substanz gemessen wird, was eine Fehlerquelle ausschliesst. Falls zwei-Waagen verwendet werden, wird unten mit Vorteil eine mindestens gleich präzise eingesetzt. Die zweite Waage kann dabei direkt das zu befüllende Zielgefäss oder einen Zwischenbehälter messen, in den die Substanz vordosiert wird.

Das erfindungsgemässe Verfahren zur Dosierung von Substanzen mit einer erfindungsgemässen Vorrichtung besteht im Wesentlichen darin, dass

a) durch Entleeren mindestens eines Substanz enthaltenden Substanzkompartiments einer Substanzaufnahmeeinrichtung Substanz in ein Gefäss dosiert wird;

b) mit einer Waage die Menge an dosierter Substanz bestimmt wird;

c) durch Steuermittel berechnet wird, ob und allenfalls wieviel Substanz noch in das Gefäss zu dosieren ist, und je nach Resultat mit Schritt a) weitergefahren wird oder die Dosierung beendet wird.

Dieses Verfahren ermöglicht ein schrittweises Annähern an die gewünschte Dosierung, das vollständig automatisch erfolgen kann. Eine besondere Geschicklichkeit des Bedieners ist nicht erforderlich.

Mit Vorteil umfasst die Substanzaufnahmeeinrichtung Substanzkompartimente verschiedener Grössenklassen und wird zunächst die grösste Anzahl Substanzkompartimente der grösstmöglichen Grössenklasse entleert, bei der noch sicher ist, dass die gewünschte Dosiermenge nicht überschritten wird, dann die grösste Anzahl Substanzkompartimente der nächstkleineren Grössenklasse, bei der noch sicher ist, dass die gewünschte Dosiermenge nicht überschritten wird, usw. bis die gewünschte Dosiermenge mit der gewünschten Genauigkeit erreicht wird. Dies ermöglicht eine raschere Annäherung an die gewünschte Dosiermenge.

Bevorzugt wird nach jeder Entleerung eines Substanzkompartiments die Menge an dosierter Substanz bestimmt. Nach jeder Entleerung mit anschliessender Gewichtsmessung kann dann die Situation aufgrund exakterer Zahlen neu eingeschätzt werden.

Alternativ wird erst nach der Entleerung mehrerer Substanzkompartimente die Menge an dosierter Substanz bestimmt. Auf diese Weise kann Zeit eingespart werden.

Bei einer bevorzugten Ausführungsvariante sind die Substanzkompartimente Röhrchen, die vor Schritt a) durch Eintauchen oder Einstechen in Substanz, die sich in einem Vorratsbehälter befindet, gefüllt werden und danach wieder aus der Substanz herausgezogen werden. Es kann so auf einfache Weise und automatisiert von den Substanzkompartimenten Substanz aufgenommen werden.

Mit Vorteil misst die Waage das sie belastende Gewicht vor und nach dem Füllen der Röhrchen und berechnen die Steuermittel daraus und aus der bekannten Geometrie der einzelnen Röhrchen die ungefähre Menge an Substanz in jedem Röhrchen. Die Dosierung kann dann basierend auf diesen Zahlen unmittelbar beginnen.

Vorteilhafterweise wird nach der ersten, vorzugsweise nach jeder, Entleerung eines Röhrchens einer Grössenklasse die ungefähre Menge an Substanz in einem Röhrchen dieser Grössenklasse neu geschätzt. Diese Kalibrierung ermöglicht eine genauere Bestimmung der Substanzmenge in einem Röhrchen einer bestimmten Grössenklasse.

Bei einer vorteilhaften Ausführungsvariante wird nach dem Füllen der Röhrchen zunächst je mindestens ein Röhrchen jeder Grössenklasse entleert und durch Bildung der Gewichtsdifferenz vor und nach der Entleerung jedes Röhrchens die ungefähre Menge an Substanz in einem Röhrchen dieser Grössenklasse bestimmt. Auch eine solche Kalibrierung ermöglicht eine genauere Bestimmung der Substanzmenge in einem Röhrchen einer bestimmten Grössenklasse.

Bevorzugt wird zunächst in einen Zwischenbehälter dosiert und bei Erreichen der gewünschten Dosiermenge mit der gewünschten Genauigkeit der Zwischenbehälter in das Gefäss entleert, während bei Überschreiten der gewünschten Dosiermenge unter Berücksichtigung der gewünschten Genauigkeit der Zwischenbehälter wieder geleert und mit der Dosierung wieder begonnen wird. Eine Überdosierung kann so auch bei sehr kleinen Gewichtstoleranzen verhindert werden.

Bei einer vorteilhaften Ausführungsvariante wird durch eine zweite Waage, an der der Zwischenbehälter befestigt ist, die tatsächliche Dosiermenge im Zwischenbehälter bestimmt. Die Dosiergenauigkeit kann so erhöht werden.

Bei einer vorteilhaften Ausführungsvariante mit n gleichen Substanzkompartimenten wird das Gewicht G der gesamthaft aufgenommenen Substanz durch eine Wägung bestimmt und aus der Anzahl der Substanzkompartimente n die Masse g pro Substanzkompartiment berechnet:

$$g = G \ / \ n \qquad (1)$$

Bei einer bevorzugten Ausführungsvariante sind die Substanzkompartimente so in Grössenklassen mit jeweils klassenweise gleichem Volumen abgestuft, dass durch Auswahl von wenigen Substanzkompartimenten verschiedener Grössenklassen der ganze Dosierbereich abgedeckt werden kann. Die Volumen der Substanzkompartimente der verschiedenen Grössenklassen stehen in einem bekannten Verhältnis zueinander. Jede der i Grössenklassen umfasst $n_i$ gleich grosse Substanzkompartimente. In diesem Fall wird die Berechnung der pro Substanzkompartiment aufgenommenen Substanzmenge nach Formel (2) durchgeführt, in der das Verhältnis des Volumens des einzelnen Substanzkompartiments zum Gesamtvolumen berücksichtigt wird. Das Gewicht der Substanz in einem Substanzkompartiment der Grössenklasse i ist $g_i$, das Volumenverhältnis wird durch den Beitrag $v_i \ / \ \Sigma(n_i \cdot v_i)$ ausgedrückt:

$$g_i = \frac{G \cdot v_i}{\sum\limits_{i=1}^{n} \left( n_i \cdot v_i \right)} \qquad (2)$$

Dabei ist zu beachten, dass sich die Ausdrücke "Volumen" und "Volumenverhältnisse" immer auf die aufgenommene bzw. aufzunehmende Substanz und nicht auf die leeren Substanzkompartimente beziehen, was für die ganze Patentanmeldung gilt. Bei einem in der Vertikalen gleichbleibenden Querschnitt sind die Volumenverhältnisse der leeren Substanzkompartimente untereinander gleich wie die der aufgenommenen Substanz. Bei einem varierenden.Querschnitt müssen die entsprechenden Verhältnisse der geometrischen Form entsprechend berechnet werden.

Die mit Substanz gefüllten Substanzkompartimente werden über das zu befüllende Gefäss gebracht und die Substanzkompartimente werden individuell (einzeln oder mehrere oder alle gleichzeitig) oder gesamthaft durch Ausstossen der aufgenommenen Substanz von oben entleert.

Ein zweiter Aspekt der Erfindung besteht im Verfahren zur Dosierung von Substanzen, das mit der erfindungsgemässen Dosiervorrichtung möglich ist.

Der Benutzer legt zunächst ein Zielband um den Sollwert fest, innerhalb dem der erreichte Wert liegen muss. Dies macht er im klassischen manuellen Verfahren bewusst oder unbewusst ebenfalls. Das kleinste mögliche Zielband entspricht der Masse der zu dosierenden Substanz in einem Substanzkompartiment der kleinsten Grössenklasse.

Sind die Substanzaufnahmeeinrichtung und die Entleereinrichtung an einer obenliegenden Waage angebracht, so ist aus dem Gesamtgewicht und dem bekannten Leergewicht der Substanzaufnahmeeinrichtung und der Entleereinrichtung das Gesamtgewicht der aufgenommenen Substanz bekannt. Da auch die Volumenverhältnisse und die Anzahl der Substanzkompartimente bekannt sind, kann nach Formel 2 das durchschnittliche Füllgewicht pro Substanzkompartiment für jede Grössenklasse berechnet werden, wobei die individuellen Füllmengen durchaus eine signifikante Streuung aufweisen können. Aufgrund des durchschnittlichen Füllgewichts kann das Steuerungsprogramm entscheiden, welche Substanzkompartimentgrösse jeweils als nächstes dosiert werden soll, bis das Zielband durch immer kleinere Zugaben erreicht ist, wobei nach jeder Zugabe gewogen wird, um die tatsächlich dosierte Menge an Substanz zu bestätigen und über die weiteren Zugaben zu entscheiden.

*Beispiel*

| | |
|---|---|
| Substanzaufnahmeeinrichtung und Entleereinrichtung leer: | 15.0000 g |
| Substanzaufnahmeeinrichtung und Entleereinrichtung nach Substanzaufnahme: | 15.0064 g |
| Aufgenommenes Gesamtgewicht G: | 6.4 mg |
| Zu dosieren (Sollwert und Zielband): | 3.32 mg $\pm$ 0.01 mg |

Berechnung der Füllmenge pro Substanzkompartiment:

1 Grössenklasse mit 10 Substanzkompartimenten, Volumen dieser Substanzkompartimente:   $V_1 = 0.1 \ \mu l$

3 Grössenklassen mit je 4 Substanzkompartimenten, Volumina der Substanzkompartimente dieser Grössenklassen:

$V_2 = 1 \ \mu l,$
$V_3 = 3 \ \mu l,$
$V_4 = 9 \ \mu l$

Nach Formel 2 werden für die verschiedenen Grössenklassen folgende durchschnittlich zu erwartende Füllmengen berechnet:

$$g_1 = G \cdot v_1 / \Sigma (n_i \cdot v_i) = 6.4 \text{ mg} \cdot 0.1 \text{ µl} / [(10 \cdot 0.1 \text{ µl}) + (4 \cdot 1 \text{ µl}) + (4 \cdot 3 \text{ µl}) + (4 \cdot 9 \text{ µl})] = 0.0121 \text{ mg}$$

$$g_2 = G \cdot v_2 / \Sigma (n_i \cdot v_i) = 6.4 \text{ mg} \cdot 1 \text{ µl} / [(10 \cdot 0.1 \text{ µl}) + (4 \cdot 1 \text{ µl}) + (4 \cdot 3 \text{ µl}) + (4 \cdot 9 \text{ µl})] = 0.121 \text{ mg}$$

$$g_3 = G \cdot v_3 / \Sigma (n_i \cdot v_i) = 6.4 \text{ mg} \cdot 3 \text{ µl} / [(10 \cdot 0.1 \text{ µl}) + (4 \cdot 1 \text{ µl}) + (4 \cdot 3 \text{ µl}) + (4 \cdot 9 \text{ µl})] = 0.362 \text{ mg}$$

$$g_4 = G \cdot v_4 / \Sigma (n_i \cdot v_i) = 6.4 \text{ mg} \cdot 9 \text{ µl} / [(10 \cdot 0.1 \text{ µl}) + (4 \cdot 1 \text{ µl}) + (4 \cdot 3 \text{ µl}) + (4 \cdot 9 \text{ µl})] = 1.09 \text{ mg}$$

Es wird dann gemäss der folgenden Tabelle dosiert:

| Zugabe | Berechnete nächste Zugabe in mg | Messung der kumulierten Zugaben in mg | Effektive letzte Zugabe in mg | Entscheid über nächste Aktion: |
|---|---|---|---|---|
| 0 | 0 | 0 | 0 | Zugabe gross |
| 1 | 1.09 | 1.003 | 1.003 | Zugabe gross |
| 2 | 1.09 | 2.385 | 1.382 | Zugabe mittel |
| 3 | 0.362 | 2.679 | 0.294 | Zugabe mittel |
| 4 | 0.362 | 3.152 | 0.473 | Zugabe klein |
| 5 | 0.121 | 3.281 | 0.129 | Zugabe feinst |
| 6 | 0.0121 | 3.291 | 0.0099 | Zugabe feinst |
| 7 | 0.0121 | 3.304 | 0.0133 | Zugabe feinst |
| 8 | 0.0121 | 3.317 | 0.0125 | Ende |

Die effektive letzte Zugabe wird jeweils entweder aufgrund der bisherigen Zugaben berechnet oder mit einer zweiten Waage gemessen.

Vorteilhafterweise wird immer mit dem gemessenen kumulierten Wert weitergearbeitet, nicht mit der berechneten Summe der einzelnen Zugaben, damit sich unvermeidbare Wägefehler nicht kumulieren.

Alternativ dazu kann (beispielsweise wenn nur eine untere Waage verwendet wird und somit das aufgenommene Gesamtgewicht nicht bekannt ist oder um Zeit zu sparen) zu Beginn des Dosiervorgangs durch das Entleeren mehrerer Substanzkompartimente mit jeweils nachfolgender Wägung die durchschnittlich abgegebene Substanzmenge pro Grössenklasse bestimmt werden, d.h. eine Kalibration vorgenommen werden. Dies kann direkt in das zu befüllende Gefäss erfolgen. Bei sehr kleinen zu dosierenden Substanzmengen kann dieser Schritt in ein Abfallgefäss erfolgen. Da dadurch die durchschnittlich zugegebene Substanzmenge pro Grössenklasse bekannt wird, kann danach durch gleichzeitiges Entleeren mehrerer Substanzkompartimente geeigneter Grösse sehr schnell bis zu einer Schwelle unterhalb der unteren Grenze des Zielbandes dosiert werden, d.h. eine Grobdosierung vorgenommen werden. Bei der nachfolgenden Feindosierung entscheidet wiederum das Steuerungsprogramm, welche Substanzkompartimentgrösse jeweils dosiert wird, bis das Zielband erreicht ist, wobei nach jeder Zugabe gewogen wird.

Bei einer vorteilhaften Ausführungsvariante wird zu einem geeigneten Zeitpunkt während des Dosiervorgangs aus den bisherigen Zugaben und/oder aus den Daten der Kalibration die effektive durchschnittliche Füllmenge und die statistische Verteilung der Substanzmengen pro Grössenklasse berechnet. Das Steuerungsprogramm kann dann den Entscheid, aus welcher Grössenklasse als nächstes dosiert wird, respektive die Schwelle, bis zu der mehrere Substanzkompartimente gleichzeitig entleert werden, dieser Verteilung anpassen. So wird auch bei sehr ungünstiger, d.h. breiter Verteilung der Füllmengen das Zielband zuverlässig erreicht und ein Überdosieren mit einer wesentlich höheren Wahrscheinlichkeit verhindert.

Im Folgenden werden die erfindungsgemässe Vorrichtung und das erfindungsgemässe Verfahren zur Dosierung von Substanzen unter Bezugnahme auf die beigefügten Zeichnungen anhand von Ausführungsbeispielen detaillierter beschrieben. Es zeigen:

Fig. 1 - eine Perspektivansicht einer Substanzaufnahmeeinrichtung und einer Entleereinrichtung eines ersten Ausführungsbeispiels der erfindungsgemässen Vorrichtung zur Dosierung von Substanzen;

Fig. 2 - eine schematische Schnittansicht der Vorrichtung von Fig. 1 mit von der Entleereinrichtung getrennter Substanzaufnahmeeinrichtung;

Fig. 3 - eine schematische Schnittansicht der voneinander getrennten Substanzaufnahmeeinrichtung und Entleereinrichtung eines zweiten Ausführungsbeispiels der erfindungsgemässen Vorrichtung;

Fig. 4 - schematisch die Aufnahme von pulverförmiger Substanz durch die Substanzaufnahmeeinrichtung von Fig. 1 durch Einstechen in in einem Vorratsbehälter vorhandene Substanz;

Fig. 5 - schematisch die Aufnahme von flüssiger Substanz durch die Substanzaufnahmeeinrichtung von Fig. 1 durch Eintauchen in in einem Vorratsbehälter vorhandene Substanz;

Fig. 6 - schematisch die Aufnahme von fester Substanz durch die Substanzaufnahmeeinrichtung von Fig. 1 durch Einstechen in einen Festkörper;

Fig. 7 - eine Schnittansicht des unteren Endes eines Substanzkompartiments in Form eines unten zugespitzten Röhrchens;

Fig. 8 - eine Schnittansicht des unteren Endes eines Substanzkompartiments in Form eines unten scharfkantig ausgebildeten Röhrchens;

Fig. 9 - eine schematische Schnittansicht der Substanzaufnahmeeinrichtung von Fig. 1 mit unterschiedlich vorgefüllten, durch Folien verschlossenen Substanzkompartimenten;

Fig. 10 - schematisch das Entleeren eines Substanzkompartiments durch einen pneumatischen Druckstoss;

Fig. 11 - schematisch das Entleeren eines Substanzkompartiments durch einen mittels einer Kolbenbewegung mechanisch induzierten Druckstoss;

Fig. 12 - schematisch einen Teil einer Entleereinrichtung und einer Substanzaufnahmeeinrichtung mit einem mit pulverförmiger Substanz gefüllten Substanzkompartiment;

Fig. 13 - schematisch das Entleeren des Substanzkompartiments von Fig. 12 durch Veränderung von dessen Geometrie durch mechanischen Druck;

Fig. 14 - schematisch die Aufnahme einer pulverförmigen Substanz durch Einstechen eines Substanzkompartiments in in einem Vorratsbehälter vorhandene Substanz;

Fig. 15 - schematisch das Anlegen einer Spannung zur Veränderung der Geometrie des Substanzkompartiments von Fig. 14 zur Verbesserung der Aufnahme von Substanz;

Fig. 16 - schematisch das Entleeren des Substanzkompartiments von Fig. 15 durch eine Änderung der angelegten Spannung zur Umkehrung der Geometrieänderung;

Fig. 17 - schematisch einen Teil einer Entleereinrichtung und einer Substanzaufnahmeeinrichtung mit einem mit flüssiger Substanz gefüllten Substanzkompartiment;

Fig. 18 - schematisch das Entleeren des Substanzkompartiments von Fig. 17 durch Veränderung der Oberflächeneigenschaften der Innenfläche des Substanzkompartiments durch Anlegen einer elektrischen Spannung;

Fig. 19 - schematisch das erste Ausführungsbeispiel der erfindungsgemässen Vorrichtung mit einer oberhalb eines zu befüllenden Gefässes angeordneten Waage, an der die Entleereinrichtung und die Substanzaufnahmeeinrichtung befestigt sind;

Fig. 20 - schematisch ein drittes Ausführungsbeispiel der erfindungsgemässen Vorrichtung mit einer unterhalb eines zu befüllenden Gefässes angeordneten Waage;

Fig. 21 - schematisch ein viertes Ausführungsbeispiel der erfindungsgemässen Vorrichtung mit einer oberhalb eines zu befüllenden Gefässes angeordneten ersten Waage, an der die Entleereinrichtung und die Substanzaufnahmeeinrichtung befestigt sind, und einer unterhalb des zu befüllenden Gefässes angeordneten zweiten Waage;

Fig. 22 - ein Flussdiagramm eines Ausführungsbeispiel des erfindungsgemässen Verfahrens zur Dosierung von Substanzen; und

Fig. 23 - eine Darstellung eines Beispiels der Dosierung einer Substanz mit dem in Fig. 22 dargestellten Verfahren.

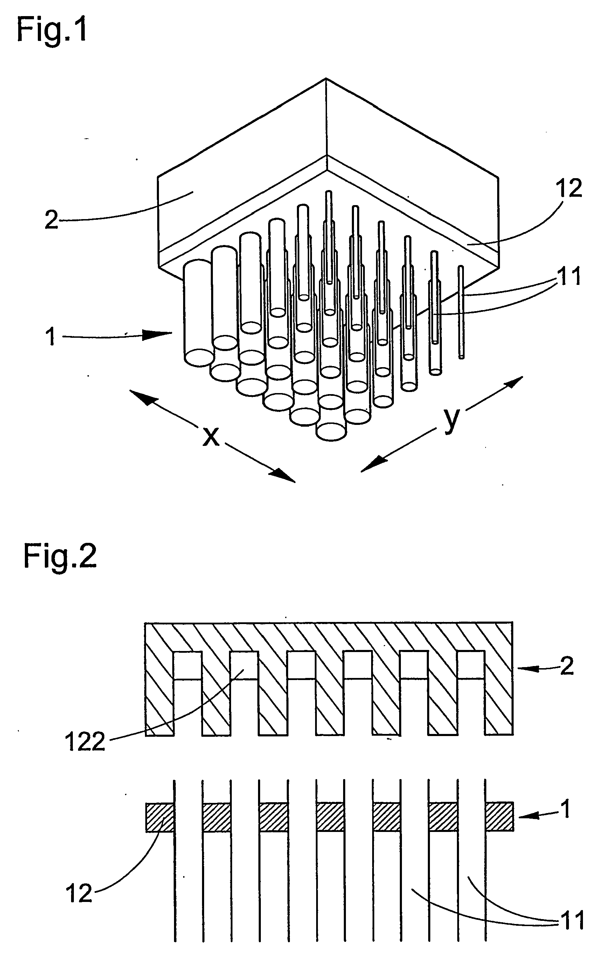

Der in den Fig. 1 und 2 dargestellte Teil eines ersten Ausführungsbeispiels der erfindungsgemässen Vorrichtung zur Dosierung von Substanzen umfasst eine Substanzaufnahmeeinrichtung 1 mit einer Trägerplatte 12, beispielsweise aus Kunststoff, an der eine Vielzahl von Substanzkompartimenten in Form von Röhrchen 11 angebracht sind, die beispielsweise aus Glas, Kunststoff oder Metall sind. Von den oben und unten offenen Röhrchen 11 liegen in sechs Grössenklassen jeweils sechs Röhrchen 11 vor. Die Röhrchen 11 einer Grössenklasse weisen den gleichen Innendurchmesser auf und sind in x-Richtung nebeneinander angeordnet. In y-Richtung nehmen die Innendurchmesser der Röhrchen 11 grössenklassenweise ab. Aufgrund der verschiedenen Innendurchmesser nehmen die Röhrchen 11 verschiedener Grössenklassen normalerweise verschiedene Mengen an zu dosierender Substanz auf.

Die Substanzaufnahmeeinrichtung 1 ist über die Trägerplatte 12 an einer Entleereinrichtung 2 abnehmbar befestigt. Die Entleereinrichtung 2 weist einen Entleermechanismus auf, mit dem die Röhrchen 11 einzeln entleert werden können. In Fig. 2 sind hiervon in vertikaler Richtung bewegbare Kolben 122 sichtbar, die in die Röhrchen 11 hineingestossen werden und dabei die Substanz in den betreffenden Röhrchen 11 aus diesen hinausdrücken. Jedem Röhrchen 11 ist ein Kolben 122 zugeordnet. Jeder Kolben 122 ist einzeln betätigbar, wobei übliche Antriebssysteme verwendet werden können.

Die Substanzaufnahmeeinrichtung 1 und die Entleereinrichtung 2 sind vorzugsweise an einem nicht gezeichneten verstellbaren Roboterarm angeordnet. Ebenfalls nicht gezeichnet ist hier die zur Vorrichtung gehörende Waage, auf die weiter unten näher eingegangen wird.

Für die gesamte weitere Beschreibung gilt folgende Festlegung. Sind in einer Figur zum Zweck zeichnerischer Eindeutigkeit Bezugszeichen enthalten, aber im unmittelbar zugehörigen Beschreibungstext nicht erwähnt, oder umgekehrt, so wird auf deren Erläuterung in vorangehenden Figurenbeschreibungen Bezug genommen.

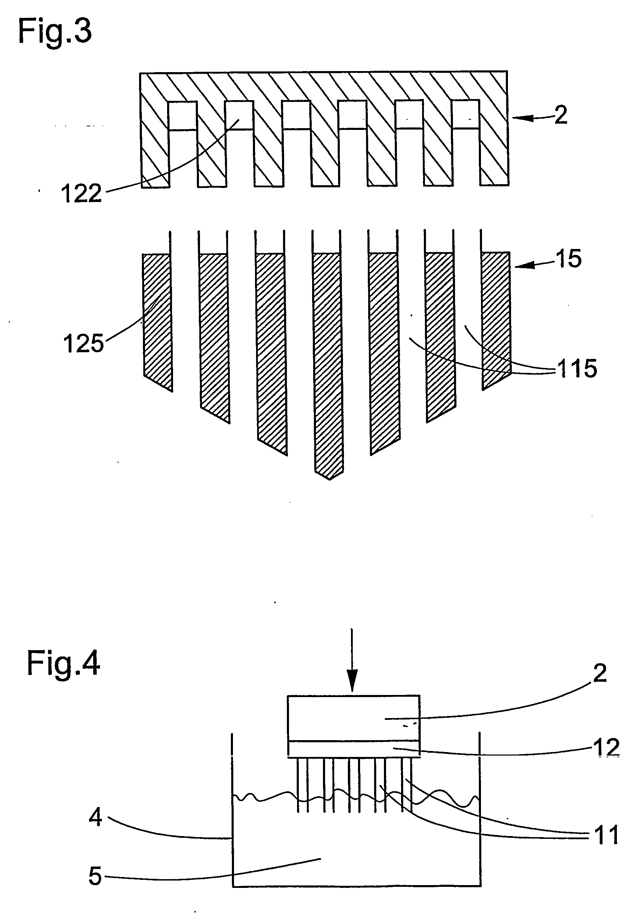

Fig. 3 zeigt die Substanzaufnahmeeinrichtung 15 und die Entleereinrichtung 2 eines zweiten Ausführungsbeispiels der erfindungsgemässen Vorrichtung, wobei die Entleereinrichtung 2 mit den Kolben 122 derjenigen des ersten Ausführungsbeispiels enspricht. Die Substanzaufnahmeeinrichtung 15 umfasst hingegen eine Substanzplatte 125, beispielsweise aus Kunststoff oder Metall, in die Substanzkompartimente 115 eingebohrt sind. Die Substanzplatte 125 ist nach unten in der Mitte zugespitzt, was ein einfacheres Eintauchen oder Einstechen in zu dosierende Substanz ermöglicht. Ausserdem ist sie leichter und kostengünstiger herstellbar als die Substanzaufnahmeeinrichtung 1 gemäss dem ersten Ausführungsbeispiel.

Fig. 4 zeigt die Aufnahme von pulverförmiger Substanz durch die Röhrchen 11 der Substanzaufnahmeeinrichtung 1 durch Einstechen in Substanz 5, die in einem Vorratsbehälter 4 vorhanden ist. Der vertikale Pfeil deutet die vertikale Verstellung der mit der Entleereinrichtung 2 verbundenen Substanzaufnahmeeinrichtung 1 zum Einstechen der Röhrchen 11 in die Substanz 5 an. Nach dem Einstechen werden die. Röhrchen 11 wieder aus der Substanz 5 herausgezogen, wobei dann aufgrund von Reibungskräften je nach Innendurchmesser der Röhrchen 11 unterschiedliche Mengen an Substanz 5 in den Röhrchen 11 aufgenommen bleiben.

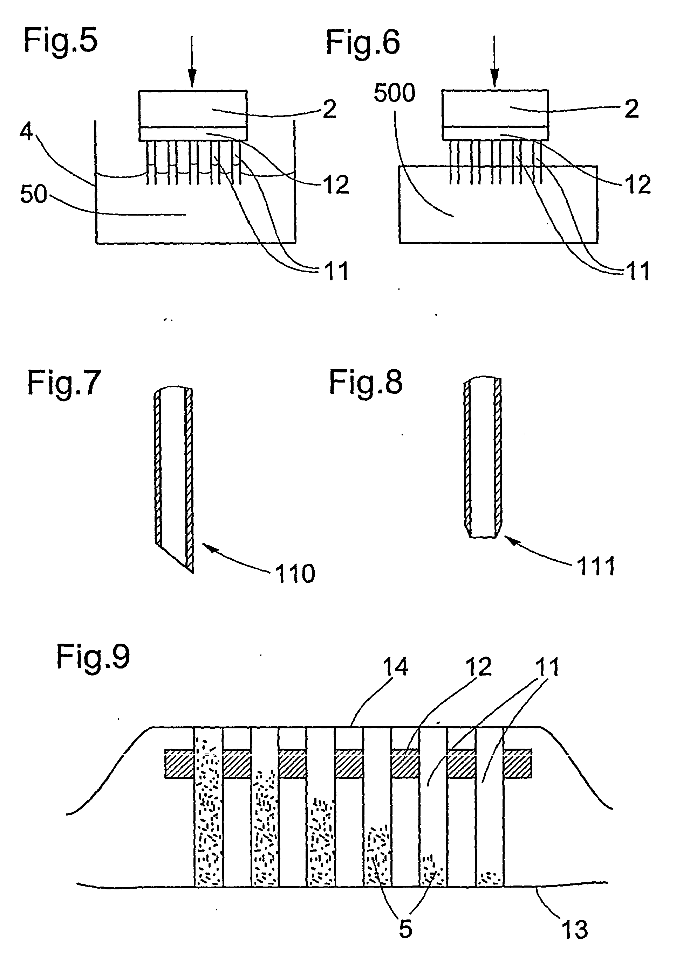

Fig. 5 zeigt die Aufnahme von flüssiger Substanz durch die Röhrchen 11 der Substanzaufnahmeeinrichtung 1 durch Eintauchen in flüssige Substanz 50, die im Vorratsbehälter 4 vorhanden ist. Der vertikale Pfeil deutet die vertikale Verstellung der mit der Entleereinrichtung 2 verbundenen Substanzaufnahmeeinrichtung 1 zum Eintauchen der Röhrchen 11 in die Substanz 50 an. Nach dem Eintauchen werden die Röhrchen 11 wieder aus der Substanz 50 herausgezogen, wobei dann aufgrund von Kapillarkräften je nach Innendurchmesser der Röhrchen 11 unterschiedliche Mengen an Substanz 50 in den Röhrchen 11 aufgenommen bleiben.

Fig. 6 zeigt die Aufnahme von fester Substanz durch die Röhrchen 11 der Substanzaufnahmeeinrichtung 1 durch Einstechen in einen Festkörper 500. Der Festkörper 500 kann aus einem fast beliebigen Material sein, das ein Einstechen der Röhrchen 11 zulässt, beispielsweise aus Polymermaterial, eine Wachsplatte oder ein Apfel. Der vertikale Pfeil deutet die vertikale Verstellung der mit der Entleereinrichtung 2 verbundenen Substanzaufnahmeeinrichtung 1 zum Einstechen der Röhrchen 11 in den Festkörper 500 an. Nach dem Einstechen werden die Röhrchen 11 wieder aus dem Festkörper 500 herausgezogen, wobei dann aufgrund von Reibungskräften je nach Innendurchmesser der Röhrchen 11 unterschiedliche Mengen an fester Substanz 500 in den Röhrchen 11 aufgenommen bleiben.

In Fig. 7 ist das untere Ende eines Substanzkompartiments in Form eines unten zugespitzten Röhrchens 110 dargestellt, während Fig. 8 das untere Ende eines Substanzkompartiments in Form eines unten scharfkantig ausgebildeten Röhrchens 111 zeigt. Diese speziellen Ausbildungen der unteren Enden der Röhrchen 110, 111 ermöglichen ein einfacheres Einstechen in pulverförmige oder feste Substanzen. Bei flüssigen Substanzen führen diese speziellen Enden zu einem regelmässigeren Loslösen von Tropfen, d.h. zu gleichmässigeren effektiven Dosiermengen von Röhrchen 110, 111 der gleichen Grössenklasse.

Die in Fig. 9 dargestellte Substanzaufnahmeeinrichtung entspricht im Wesentlichen derjenigen von Fig. 1, die Röhrchen 11 sind aber bereits mit grössenklassenweise unterschiedlichen Mengen an Substanz 5 vorgefüllt. Die Röhrchen 11 sind oben und unten durch Folien 14 bzw. 13 verschlossenen, so dass die Substanzaufnahmeeinrichtung problemlos transportiert und gelagert werden kann, ohne dass Substanz 5 verloren geht oder verschmutzt wird. Die Folien 13, 14 können unmittelbar vor der Verwendung als ganzes abgezogen werden oder sie werden alternativ beim Entleeren der Substanzkompartimente 11 zerstört, wobei vorzugsweise solche Folien 13, 14 verwendet werden, die so zerrissen werden, dass keine Folienrückstände in die zu befüllenden Gefässe fallen.

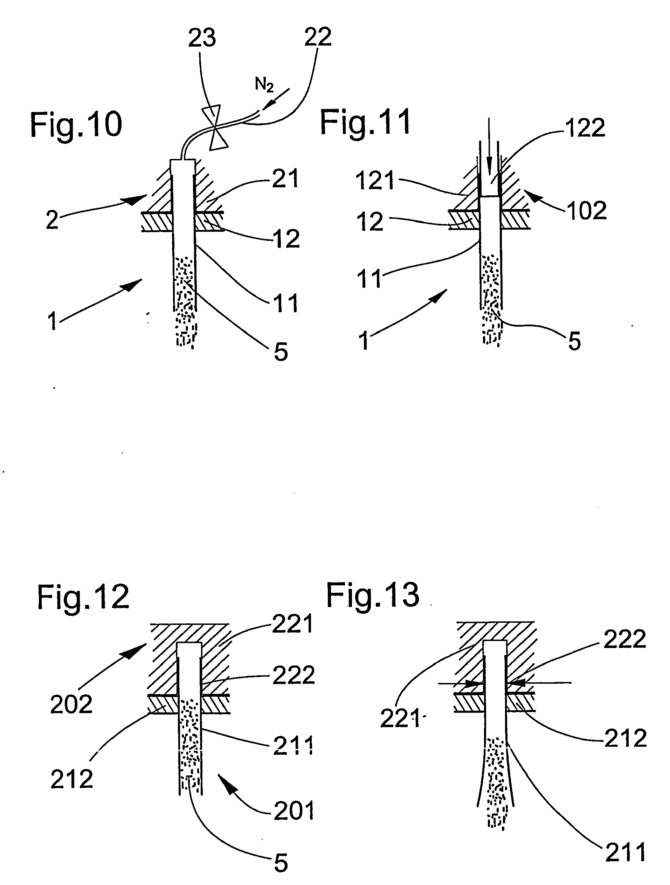

Bei dem in Fig. 10 dargestellten Ausführungsbeispiel erfolgt das Entleeren eines Röhrchens 11 durch einen pneumatischen Druckstoss. Hierzu wird über eine Gasleitung 22 Stickstoff unter Druck in das Röhrchen 11 eingelassen, was zu einem Druckstoss führt, welcher die aufgenommene Substanz 5 unten aus dem Röhrchen 11 hinausdrückt. Der Stickstoff stammt beispielsweise aus einem nicht dargestellten Stickstoffbehälter und sein Einlass in das Röhrchen 11 wird durch Öffnen eines in der Gasleitung 22 angeordneten Ventils 23 gestartet.

Bei dem in Fig. 11 dargestellten Ausführungsbeispiel erfolgt das Entleeren eines Röhrchens 11 durch einen mechanisch induzierten Druckstoss. Hierzu weist die Entleereinrichtung 102 eine Zylinderplatte 121 auf, die mit einer Vielzahl von vertikalen zylindrischen Bohrungen versehen ist, in denen jeweils ein Kolben 122 vertikal verschiebbar ist. Pro Röhrchen 11 ist ein Kolben 122 vorhanden, wobei die Kolben 122 unabhängig voneinander mittels herkömmlichen Antrieben einzeln bewegbar sind.

Bei dem in den Fig. 12 und 13 dargestellten Ausführungsbeispiel ist ein Röhrchen 211 einer Substanzaufnahmeeinrichtung 201 mit pulverförmiger Substanz 5 gefüllt. Das Röhrchen 211 weist flexible Wände auf, deren Geometrie durch Ausübung eines mechanischen Drucks verändert werden kann. Zur Ausübung eines mechanischen Drucks auf das Röhrchen 211 weist eine Entleereinrichtung 202, an der die Substanzaufnahmeeinrichtung 201 über eine Trägerplatte 212 befestigt ist, in Bohrungen in einer Basisplatte 221 Druckelemente 222 auf, beispielsweise Piezolemente, insbesondere piezokeramische Verbundelemente. Im vorliegenden Fall wird das Röhrchen 211 durch Ausübung eines mechanischen Drucks auf seinen oberen Teil unten aufgeweitet, was zu einer Loslösung der aufgenommenen Substanz 5 und somit zur Entleerung des Röhrchens 211 führt.

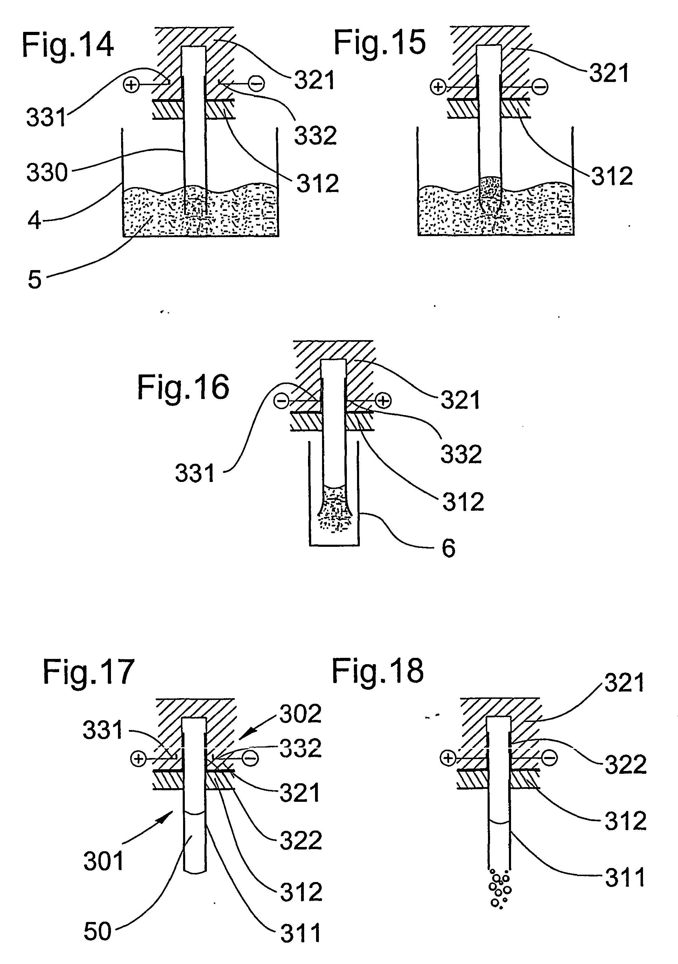

Die Fig. 14 und 15 zeigen das Aufnehmen einer pulverförmigen Substanz 5 durch Einstechen eines Röhrchens 330 einer Substanzaufnahmeeinrichtung in die im Vorratsbehälter 4 vorhandene Substanz 5. Nach dem Einstechen in die Substanz 5 wird der untere Teil des Röhrchens 330, das zumindest in seinem unteren Teil flexible Wände aufweist, durch Anlegen einer elektrischen Spannung an seinen oberen Teil verengt, wodurch die im Röhrchen 330 vorhandene Substanz 5 verdichtet und festgeklemmt wird. Das Anlegen der elektrischen Spannung erfolgt mittels einer Entleereinrichtung, an der die Substanzaufnahmeeinrichtung über eine Trägerplatte 312 befestigt ist. Die Entleereinrichtung weist hierzu Spannungselektroden 331 und 332 auf, die in einer Basisplatte 321 derart gelagert sind, dass sie mit dem Röhrchen 330 in Kontakt bringbar sind. Damit sich die Geometrie des unteren Teils des Röhrchens 330 aufgrund des Anlegens einer Spannung im oberen Teil ändert, sind am unteren Teil beispielsweise Piezolemente, insbesondere piezokeramische Verbundelemente, angebracht, die in elektrisch leitender Verbindung mit den Bereichen des Röhrchens 330 sind, an denen die Spannung angelegt wird.

Nach dem Herausziehen des Röhrchens 330 aus dem Vorratsbehälter 4 kann es beispielsweise mittels eines Roboterarms, an dem die Entleereinrichtung und die Substanzaufnahmeeinrichtung angebracht sein können, von oben in ein Gefäss 6, in das die Substanz 5 zu dosieren ist, eingeführt werden. Wie in Fig. 16 dargestellt, wird die angelegte elektrischen Spannung dann umgepolt, was bei geeigneter Ausbildung des Röhrchens 330 und der Piezoelemente zu einer Aufweitung des unteren Teils des Röhrchens 330 und zu einer Entleerung des Röhrchens 330 führt.

Bei dem in den Fig. 17 und 18 dargestellten Ausführungsbeispiel weist eine Entleereinrichtung 302 ebenfalls Spannungselektroden 331 und 332 auf, die in einer Basisplatte 321 derart gelagert sind, dass sie mit einem flüssige Substanz 50 enthaltenden Röhrchen 311 in Kontakt bringbar sind. Die Entleereinrichtung 302 kann aber zusätzlich oder alternativ auch Temperiermittel 322, beispielsweise eine elektrische Widerstandsheizung, aufweisen, mit denen die Temperatur des Röhrchens 311 verändert werden kann. Auch hier ist die das Röhrchen 311 enthaltende Substanzaufnahmeeinrichtung 301 über die Trägerplatte 312 mit der Entleereinrichtung 302 verbunden.

Durch Anlegen einer elektrischen Spannung mit den Spannungselektroden 331 und 332 und/oder durch Verändern der Temperatur des Röhrchens 311 können die Oberflächeneigenschaften der Innenfläche des Röhrchens 311 geändert und auf diese Weise eine Entleerung des Röhrchens 311 ausgelöst werden. Damit die Oberflächeneigenschaften im gewünschten Sinne geändert werden, kann die Innenfläche des Röhrchens 311 beispielsweise mit einem Halbleiter beschichtet sein, der durch das Anlegen einer elektrischen Spannung von einem isolierenden Zustand in einen leitenden Zustand übergeht. Dadurch ändert sich die Benetzbarkeit der Innenfläche des Röhrchens 311, was die Entleerung auslösen kann.

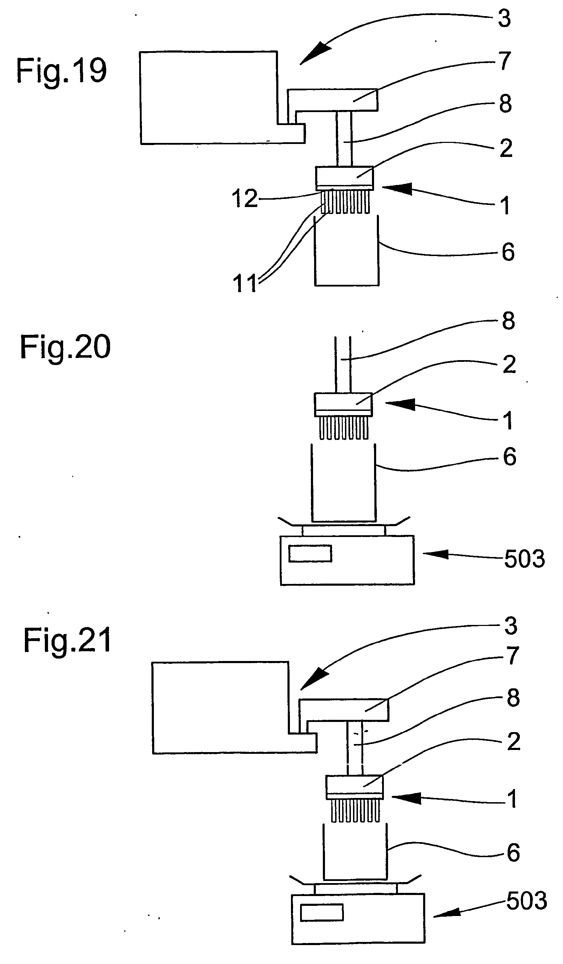

Fig. 19 zeigt schematisch das erste Ausführungsbeispiel der erfindungsgemässen Vorrichtung mit einer oberhalb eines zu befüllenden Gefässes 6 angeordneten Waage 3, an der die Entleereinrichtung 2 und die Substanzaufnahmeeinrichtung 1 über Koppelelemente 7, 8 angebracht sind. Die Waage 3 ist beispielsweise wie in der WO 02/29369 beschrieben aufgebaut, auf die an dieser Stelle explizit verwiesen wird, und die Befestigung der Substanzaufnahmeeinrichtung 1, Entleereinrichtung 2 und Koppelelemente 7, 8 kann auf äquivalente Weise erfolgen.

Fig. 20 zeigt schematisch ein drittes Ausführungsbeispiel der erfindungsgemässen Vorrichtung mit einer unterhalb des zu befüllenden Gefässes 6 angeordneten Waage 503, welche eine herkömmliche Waage sein kann.

Bei dem in Fig. 21 dargestellten vierten Ausführungsbeispiel der erfindungsgemässen Vorrichtung ist oberhalb des zu befüllenden Gefässes 6 die erste Waage 3 angeordnet, an der die Entleereinrichtung 2 und die Substanzaufnahmeeinrichtung 1 über die Koppelelemente 7, 8 angebracht sind, während unterhalb des zu befüllenden Gefässes 6 die zweite Waage 503 angeordnet ist. Es kann so sowohl die von der Substanzaufnahmeeinrichtung 1 abgegebene Menge an Substanz als auch die im Gefäss 6 sich ansammelnde Menge an Substanz gemessen werden.

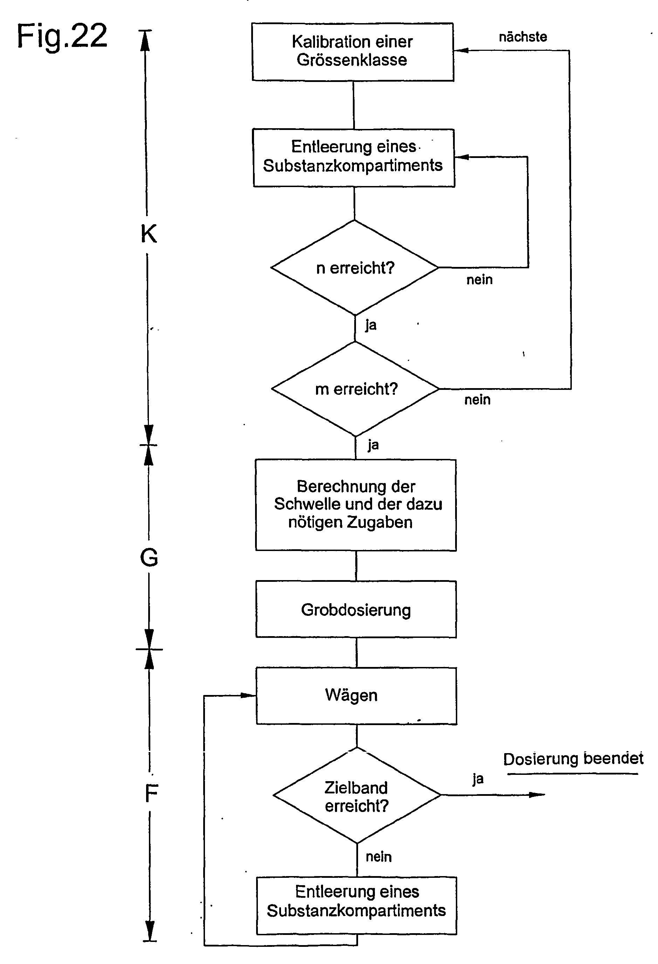

Fig. 22 zeigt ein Flussdiagramm eines Ausführungsbeispiels des erfindungsgemässen Verfahrens zur Dosierung von Substanzen. Zunächst werden der zu erreichende Sollwert und in Abhängigkeit der zur Verfügung stehenden Substanzkompartimente das sich aus der gewünschten Genauigkeit ergebende Zielband festgelegt (im Flussdiagramm nicht dargestellt).

Danach findet eine Kalibration K der Substanzkompartimente der hier m verschiedenen Grössenklassen statt. Dazu werden nacheinander n Substanzkompartimente einer Grössenklasse entleert und jeweils nach jeder Entleerung wird gewogen. Hieraus kann die durchschnittliche Substanzmenge in einem Substanzkompartiment dieser Grössenklasse berechnet werden. Alle m Grössenklassen werden nacheinander kalibriert.

Nach der Kalibration K findet eine Grobdosierung G statt. Dabei wird zunächst eine Schwelle berechnet, bis zu der ohne grosse Gefahr einer Überdosierung grobdosiert werden kann. Nach der Schwellenberechnung erfolgt die Berechnung der zur Erreichung der Schwelle noch benötigten Substanzzugaben. Die berechneten Substanzzugaben werden dann durch Entleerung der entsprechenden Anzahl Substanzkompartimente in das zu befüllende Gefäss vorgenommen.

In der nachfolgenden Feindosierung F wird zunächst gewogen, wieviel in das zu befüllende Gefäss dosiert worden ist. Das Messresultat wird dann mit dem Zielband verglichen. Liegt das Messresultat im Zielband ist die Dosierung beendet. Liegt es unterhalb des Zielbands, wird ein weiteres geeignetes Substanzkompartiment entleert und wieder gewogen etc. bis schliesslich das Zielband erreicht wird.

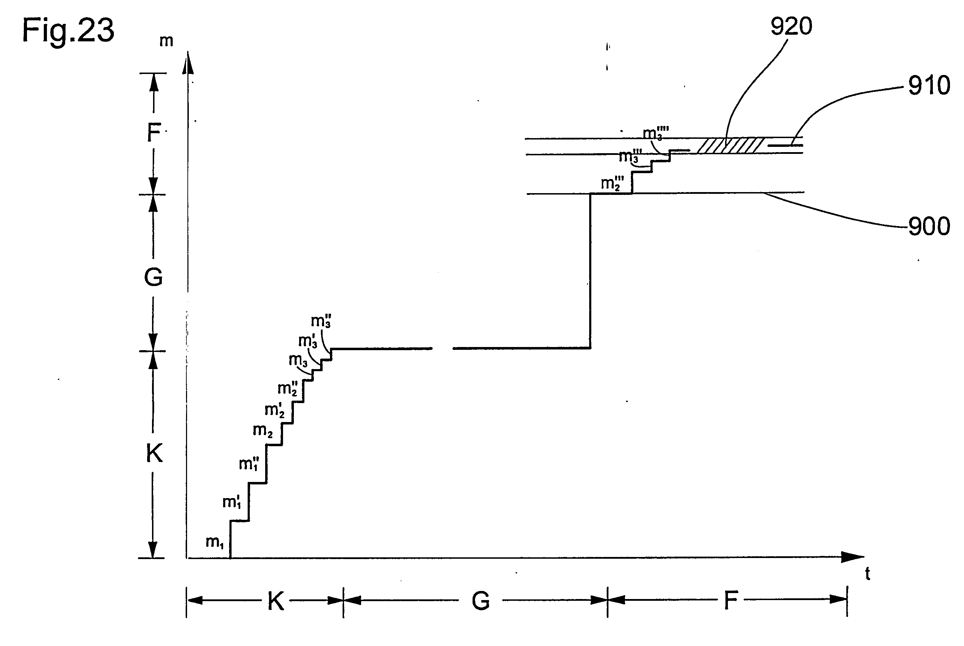

In Fig. 23 ist ein Beispiel der Dosierung einer Substanz mit dem oben beschriebenen Verfahren dargestellt. Zunächst werden der Sollwert 910 und das Zielband 920 festgelegt. Danach werden die m = 3 verschiedenen Grössenklassen von Substanzkompartimenten nacheinander kalibriert, wobei für jede Grössenklasse n = 3 Entleerungen und Wägungen vorgenommen werden. Hieraus wird dann für jede Grössenklasse die durchschnittliche Substanzmenge in einem Substanzkompartiment berechnet.

In der nachfolgenden Grobdosierung G wird zunächst die Schwelle 900 berechnet, bis zu der ohne grosse Gefahr einer Überdosierung grobdosiert werden kann. Nach der Schwellenberechnung erfolgt die Berechnung der zur Erreichung der Schwelle noch benötigten Substanzzugaben und diese werden anschliessend durch Entleerung der entsprechenden Anzahl Substanzkompartimente in das zu befüllende Gefäss vorgenommen.

Schliesslich erfolgt die Feindosierung F, in der zunächst gewogen wird, wieviel in das zu befüllende Gefäss dosiert worden ist. Im vorliegenden Fall hat sich gezeigt, dass das Zielband 920 noch nicht erreicht worden ist, und es wird ein Substanzkompartiment der zweiten Grössenklasse entleert, nochmals gewogen, wieder mit dem Zielband 920 verglichen, noch ein Substanzkompartiment der dritten Grössenklasse entleert, nochmals gewogen, wieder mit dem Zielband 920 verglichen, nochmals ein Substanzkompartiment der dritten Grössenklasse entleert, nochmals gewogen und wieder mit dem Zielband 920 verglichen. Es konnte dann festgestellt werden, dass das Messresultat im Zielband liegt, und die Dosierung konnte beendet werden.

**Patentansprüche**

1. Vorrichtung zur Dosierung von Substanzen, mit einer Substanzaufnahmeeinrichtung (1; 15; 201; 301), die mindestens ein Substanzkompartiment (11; 110; 111; 115; 211; 311; 330) zur Aufnahme von zu dosierender Substanz (5; 50; 500) umfasst, einer Entleereinrichtung (2; 102; 202; 302) zur Entleerung des mindestens einen Substanzkompartiments (11; 110; 111; 115; 211; 311; 330) und einer Waage (3; 503) zur Bestimmung der Menge an dosierter Substanz (5; 50; 500), **dadurch gekennzeichnet, dass** die Substanzaufnahmeeinrichtung (1; 15; 201; 301) eine Vielzahl von Substanzkompartimenten (11; 110; 111; 115; 211; 311; 330) umfasst, die einzeln entleerbar sind, und die Vorrichtung ausserdem Steuermittel umfasst, die die Entleerung der Substanzkompartimente (11; 110; 111; 115; 211; 311; 330) in Abhängigkeit von der mittels der Waage (3; 503) bestimmten Menge an dosierter Substanz (5; 50; 500) steuern.

2. Vorrichtung nach Anspruch 1, **dadurch gekennzeichnet, dass** die Substanzaufnahmeeinrichtung (1; 15; 201; 301) Substanzkompartimente (11; 110; 111; 115; 211; 311; 330) verschiedener Grössenklassen umfasst, mit denen verschiedene Mengen an zu dosierender Substanz (5; 50; 500) aufnehmbar sind.

3. Vorrichtung nach Anspruch 2, **dadurch gekennzeichnet, dass** zumindest einige der Grössenklassen über mindestens einen Faktor 5 abgestuft sind, vorzugsweise im Verhältnis 1:2:5.

4. Vorrichtung nach einem der Ansprüche 1 bis 3, **dadurch gekennzeichnet, dass** mindestens einige der Substanzkompartimente (11; 110; 111; 115; 211; 311; 330) mit zu dosierender Substanz (5; 50; 500) vorgefüllt und vorzugsweise verschlossen sind.

5. Vorrichtung nach einem der Ansprüche 1 bis 4, **dadurch gekennzeichnet, dass** die Substanzkompartimente durch vertikal angeordnete Röhrchen (11; 110; 111; 211; 311; 330) gebildet sind.

6. Vorrichtung nach Anspruch 5, **dadurch gekennzeichnet, dass** die Röhrchen (11; 110; 111; 211; 311; 330) grössenklassenweise verschiedene Innendurchmesser aufweisen.

7. Vorrichtung nach Anspruch 5 oder 6, **dadurch gekennzeichnet, dass** die Innendurchmesser der Röhrchen (11; 110; 111; 211; 311; 330) kleiner als 5 mm, vorzugsweise kleiner als 1 mm, bevorzugter kleiner als 0,5 mm, noch bevorzugter kleiner als 0,1 mm, sind.

8. Vorrichtung nach einem der Ansprüche 5 bis 7, **dadurch gekennzeichnet, dass** mindestens einige der Röhrchen sich von oben nach unten verjüngen.

9. Vorrichtung nach einem der Ansprüche 5 bis 8, **dadurch gekennzeichnet, dass** mindestens einige der Röhrchen (110; 111) unten zugespitzt oder scharfkantig ausgebildet sind.

10. Vorrichtung nach einem der Ansprüche 5 bis 9, **dadurch gekennzeichnet, dass** mindestens einige der Röhrchen (11; 110; 111; 211; 311; 330) mit zu dosierender Substanz (5; 50; 500) vorgefüllt und vorzugsweise die beiden Enden der Röhrchen (11; 110; 111; 211; 311; 330) mit einer Folie (13, 14) verschlossen sind.

11. Vorrichtung nach einem der Ansprüche 1 bis 10, **dadurch gekennzeichnet, dass** mindestens einige der Substanzkompartimente (11; 110; 111; 115; 211; 311; 330) eine Innenfläche mit einem arithmetischen Mittenrauhwert $R_a$ grösser als 0,5 $\mu$m aufweisen.

12. Vorrichtung nach einem der Ansprüche 1 bis 11, **dadurch gekennzeichnet, dass** sie verschiedene Klassen von Substanzkompartimenten (11; 110; 111; 115; 211; 311; 330) mit Innenflächen mit unterschiedlichen arithmetischen Mittenrauhwerten $R_a$ aufweist.

13. Vorrichtung nach einem der Ansprüche 1 bis 12, **dadurch gekennzeichnet, dass** mindestens einige der Substanzkompartimente (11; 110; 111; 115; 211; 311; 330) an ihrer Innenfläche flexible Lamellen und/oder Widerhaken aufweisen.

14. Vorrichtung nach einem der Ansprüche 1 bis 13, **dadurch gekennzeichnet, dass** sie verschiedene Klassen von Substanzkompartimenten (11; 110; 111; 115; 211; 311; 330) mit Innenflächen mit unterschiedlicher Benetzbarkeit aufweist.

**15.** Vorrichtung nach einem der Ansprüche 1 bis 14, **dadurch gekennzeichnet, dass** die Substanzaufnahmeeinrichtung (1; 15; 201; 301) von der Entleereinrichtung (2; 102; 202; 302) automatisch lösbar ist.

**16.** Vorrichtung nach einem der Ansprüche 1 bis 15, **dadurch gekennzeichnet, dass** die Substanzkompartimente (11; 110; 111; 211; 311; 330) in der Substanzaufnahmeeinrichtung (1; 201; 301) einzeln montiert sind und ihre Anzahl varierbar ist.

**17.** Vorrichtung nach einem der Ansprüche 1 bis 16, **dadurch gekennzeichnet, dass** die Substanzkompartimente (11; 110; 111; 211; 311; 330) in der Substanzaufnahmeeinrichtung (1; 201; 301) einzeln zwischen einer Füllposition, in der sie befüllbar sind, und einer inaktiven Position, in der sie nicht befüllbar sind, verstellbar montiert sind.

**18.** Vorrichtung nach einem der Ansprüche 1 bis 17, **dadurch gekennzeichnet, dass** sie Mittel zur vertikalen Verstellung der Substanzaufnahmeeinrichtung (1; 15; 201; 301) umfasst.

**19.** Vorrichtung nach einem der Ansprüche 1 bis 18, **dadurch gekennzeichnet, dass** die Entleereinrichtung (2) Mittel (22, 23) zur Beaufschlagung jedes einzelnen Substanzkompartiments (11) mit Druckgas umfasst.

**20.** Vorrichtung nach einem der Ansprüche 1 bis 19, **dadurch gekennzeichnet, dass** die Entleereinrichtung (102) für jedes Substanzkompartiment (11) einen verstellbaren Kolben (122) aufweist.

**21.** Vorrichtung nach einem der Ansprüche 1 bis 20, **dadurch gekennzeichnet, dass** die Entleereinrichtung (202) Mittel zur Veränderung der Geometrie jedes einzelnen Substanzkompartiments (211; 330) aufweist, die vorzugsweise Mittel (222; 331, 332) zur Erzeugung eines mechanischen Drucks, einer elektrischen Spannung oder einer Temperaturänderung umfassen.

**22.** Vorrichtung nach einem der Ansprüche 1 bis 21, **dadurch gekennzeichnet, dass** die Entleereinrichtung (302) Mittel zur Veränderung der Oberflächeneigenschaften der Innenfläche jedes einzelnen Substanzkompartiments (311) aufweist, die vorzugsweise Mittel (322; 331, 332) zur Erzeugung einer elektrischen Spannung und/oder einer Temperaturänderung umfassen.

**23.** Vorrichtung nach einem der Ansprüche 1 bis 22, **dadurch gekennzeichnet, dass** die Entleereinrichtung Mittel zur Veränderung der Fliesseigenschaften der zu dosierenden Substanz (5; 50; 500) in jedem einzelnen Substanzkompartiment aufweist, die vorzugsweise Mittel zur Erzeugung einer elektrischen Spannung oder einer Temperaturänderung umfassen.

**24.** Vorrichtung nach einem der Ansprüche 1 bis 23, **dadurch gekennzeichnet, dass** die Entleereinrichtung (2; 102; 202; 302) und die Substanzaufnahmeeinrichtung (1; 15; 201; 301) an der Waage (3) angeordnet sind, so dass sie von dieser gewogen werden.

**25.** Vorrichtung nach einem der Ansprüche 1 bis 24, **dadurch gekennzeichnet, dass** die Waage (503) bzw. eine zweite Waage (503) ausgebildet ist, um ein zu befüllendes Gefäss (6) aufzunehmen und das Gewicht des Gefässes (6) und der in das Gefäss (6) dosierten Substanz (5; 50; 500) zu messen.

**26.** Verfahren zur Dosierung von Substanzen mit einer Vorrichtung nach einem der Ansprüche 1 bis 25, **dadurch gekennzeichnet, dass**

a) durch Entleeren mindestens eines Substanz (5; 50; 500) enthaltenden Substanzkompartiments (11; 110; 111; 115; 211; 311; 330) einer Substanzaufnahmeeinrichtung (1; 15; 201; 301) Substanz (5; 50; 500) in ein Gefäss (6) dosiert wird;
b) mit einer Waage (3; 503) die Menge an dosierter Substanz (5; 50; 500) bestimmt wird;
c) durch Steuermittel berechnet wird, ob und allenfalls wieviel Substanz (5; 50; 500) noch in das Gefäss (6) zu dosieren ist, und je nach Resultat mit Schritt a) weitergefahren wird oder die Dosierung beendet wird.

**27.** Verfahren nach Anspruch 26, **dadurch gekennzeichnet, dass** die Substanzaufnahmeeinrichtung (1; 15; 201; 301) Substanzkompartimente (11; 110; 111; 115; 211; 311; 330) verschiedener Grössenklassen umfasst und zunächst die grösste Anzahl Substanzkompartimente (11; 110; 111; 115; 211; 311; 330) der grösstmöglichen Grössenklasse entleert wird, bei der noch sicher ist, dass die gewünschte Dosiermenge nicht überschritten wird, dann die grösste Anzahl Substanzkompartimente (11; 110; 111; 115; 211; 311; 330) der nächstkleineren Grössenklasse, bei der noch sicher ist, dass die gewünschte Dosiermenge nicht überschritten wird, usw. bis die gewünschte Dosiermenge mit der gewünschten Genauigkeit erreicht wird.

28. Verfahren nach Anspruch 26 oder 27, **dadurch gekennzeichnet, dass** nach jeder Entleerung eines Substanzkompartiments (11; 110; 111; 115; 211; 311; 330) die Menge an dosierter Substanz (5; 50; 500) bestimmt wird.

29. Verfahren nach Anspruch 26 oder 27, **dadurch gekennzeichnet, dass** erst nach der Entleerung mehrerer Substanzkompartimente (11; 110; 111; 115; 211; 311; 330) die Menge an dosierter Substanz (5; 50; 500) bestimmt wird.

30. Verfahren nach einem der Ansprüche 26 bis 29, **dadurch gekennzeichnet, dass** die Substanzkompartimente (11; 110; 111; 115; 211; 311; 330) vor Schritt a) durch Eintauchen oder Einstechen in Substanz (5; 50; 500), die sich in einem Vorratsbehälter (4) befindet, gefüllt werden und danach wieder aus der Substanz (5; 50; 500) herausgezogen werden.

31. Verfahren nach Anspruch 30, **dadurch gekennzeichnet, dass** die Waage (3) das sie belastende Gewicht vor und nach dem Füllen der Substanzkompartimente (11; 110; 111; 115; 211; 311; 330) misst und die Steuermittel daraus und aus der bekannten Geometrie der einzelnen Substanzkompartimente (11; 110; 111; 115; 211; 311; 330) die ungefähre Menge an Substanz (5; 50; 500) in jedem Substanzkompartiment (11; 110; 111; 115; 211; 311; 330) berechnen.

32. Verfahren nach Anspruch 30 oder 31, **dadurch gekennzeichnet, dass** nach der ersten, vorzugsweise nach jeder, Entleerung eines Substanzkompartiments (11; 110; 111; 115; 211; 311; 330) einer Grössenklasse die ungefähre Menge an Substanz (5; 50; 500) in einem Substanzkompartiment (11; 110; 111; 115; 211; 311; 330) dieser Grössenklasse neu geschätzt wird.

33. Verfahren nach einem der Ansprüche 30 bis 32, **dadurch gekennzeichnet, dass** nach dem Füllen der Substanzkompartimente (11; 110; 111; 115; 211; 311; 330) zunächst je mindestens ein Substanzkompartiment (11; 110; 111; 115; 211; 311; 330) jeder Grössenklasse entleert wird und durch Bildung der Gewichtsdifferenz vor und nach der Entleerung jedes Substanzkompartiments (11; 110; 111; 115; 211; 311; 330) die ungefähre Menge an Substanz (5; 50; 500) in einem Substanzkompartiment (11; 110; 111; 115; 211; 311; 330) dieser Grössenklasse bestimmt wird.

34. Verfahren nach einem der Ansprüche 26 bis 33, **dadurch gekennzeichnet, dass** zunächst in einen Zwischenbehälter dosiert wird und bei Erreichen der gewünschten Dosiermenge mit der gewünschten Genauigkeit der Zwischenbehälter in das Gefäss (6) entleert wird, während bei Überschreiten der gewünschten Dosiermenge unter Berücksichtigung der gewünschten Genauigkeit der Zwischenbehälter wieder geleert und mit der Dosierung wieder begonnen wird.

35. Verfahren nach Anspruch 34, **dadurch gekennzeichnet, dass** durch eine zweite Waage, an der der Zwischenbehälter befestigt ist, die tatsächliche Dosiermenge im Zwischenbehälter bestimmt wird.

## Claims

1. Device for dosage of substances, with a substance intake portion (1; 15; 201; 301), which comprises at least one substance compartment (11; 110; 111; 115; 211; 311; 330) for the intake of substance (5; 50; 500) to be dosed, an emptying portion (2; 102; 202; 302) for the emptying of the at least one substance compartment (11; 110; 111; 115; 211; 311; 330) and a weighing balance (3; 503) for the determination of the quantity of dosed substance (5; 50; 500), **characterised in that** the substance intake portion (1; 15; 201; 301) comprises a plurality of substance compartments (11; 110; 111; 115; 211; 311; 330), which are individually emptiable, and the device comprises in addition control means, which control the emptying of the substance compartments (11; 110; 111; 115; 211; 311; 330) in a manner dependent on the quantity of dosed substance (5; 50; 500), which is determined by means of the weighing balance (3; 503).

2. Device according to claim 1, **characterised in that** the substance intake portion (1; 15; 201; 301) comprises substance compartments (11; 110; 111; 115; 211; 311; 330) of various size classes, with which various quantities of substance (5; 50; 500) to be dosed can be intaken.

3. Device according to claim 2, **characterised in that** least some of the size classes are graduated across at least a factor of 5, preferably in the ratio 1:2:5.

4. Device according to any one of claims 1 to 3, **characterised in that** at least some of the substance compartments (11; 110; 111; 115; 211; 311; 330) are pre-filled with substance (5; 50; 500) to be dosed and preferably are sealed.

5. Device according to any one of claims 1 to 4, **characterised in that** the substance compartments are formed by vertically arranged tubes (11; 110; 111; 211; 311; 330).

6. Device according to claim 5, **characterised in that** the tubes (11; 110; 111; 211; 311; 330) of different size classes have different inner diameters.

7. Device according to claim 5 or 6, **characterised in that** the inner diameters of the tubes (11; 110; 111; 211; 311; 330) are smaller than 5 mm, preferably smaller than 1 mm, more preferably smaller than 0.5 mm, in particular preferably smaller than 0.1 mm.

8. Device according to any one of claims 5 to 7, **characterised in that** at least some of the tubes narrow from the top to the bottom.

9. Device according to any one of claims 5 to 8, **characterised in that** at least some of the tubes (110; 111) have pointed or sharp-edged lower sections.

10. Device according to any one of claims 5 to 9, **characterised in that** at least some of the tubes (11; 110; 111; 211; 311; 330) are pre-filled with substance (5; 50; 500) to be dosed and preferably the two ends of the tubes (11; 110; 111; 211; 311; 330) are sealed with a foil (13, 14).

11. Device according to any one of claims 1 to 10, **characterised in that** at least some of the substance compartments (11; 110; 111; 115; 211; 311; 330) have an inner surface with an arithmetic mean roughness value $R_a$ larger than 0.5 $\mu$m.

12. Device according to any one of claims 1 to 11, **characterised in that** it comprises various classes of substance compartments (11; 110; 111; 115; 211; 311; 330) with inner surfaces with different arithmetic mean roughness values $R_a$.

13. Device according to any one of claims 1 to 12, **characterised in that** at least some of the substance compartments (11; 110; 111; 115; 211; 311; 330) have on their inner surface flexible lamellae and/or barbs.

14. Device according to any one of claims 1 to 13, **characterised in that** it comprises various classes of substance compartments (11; 110; 111; 115; 211; 311; 330) with inner surfaces with different wettability.

15. Device according to any one of claims 1 to 14, **characterised in that** the substance intake portion (1; 15; 201; 301) is automatically removable from the emptying portion (2; 102; 202; 302).

16. Device according to any one of claims 1 to 15, **characterised in that** the substance compartments (11; 110; 111; 211; 311; 330) are individually mounted in the substance intake portion (1; 201; 301) and their number is variable.

17. Device according to any one of claims 1 to 16, **characterised in that** the substance compartments (11; 110; 111; 211; 311; 330) in the substance intake portion (1; 201; 301) are individually displaceably mounted between a fill position, in which they are fillable, and an inactive position, in which they are not fillable.

18. Device according to any one of claims 1 to 17, **characterised in that** it comprises means for vertical displacement of the substance intake portion (1; 15; 201; 301).

19. Device according to any one of claims 1 to 18, **characterised in that** the emptying portion (2) comprises means (22, 23) for the admission of pressure gas into every individual substance compartment (11).

20. Device according to any one of claims 1 to 19, **characterised in that** for every substance compartment (11) the emptying portion (102) has a displaceable piston (122).

**21.** Device according to any one of claims 1 to 20, **characterised in that** the emptying portion (202) has means for the alteration of the geometry of every individual substance compartment (211; 330), which preferably comprise means (222; 331, 332) for the production of a mechanical pressure, a voltage or a temperature change.

**22.** Device according to any one of claims 1 to 21, **characterised in that** the emptying portion (302) has means for the alteration of the surface properties of the inner surface of every individual substance compartment (311), which preferably comprise means (322, 331, 332) for the production of a voltage and/or a temperature change.

**23.** Device according to any one of claims 1 to 22, **characterised in that** the emptying portion has means for the alteration of the flow properties of the substance to be dosed (5; 50; 500) in every individual substance compartment, which preferably comprise means for the production of a voltage or a temperature change.

**24.** Device according to any one of claims 1 to 23, **characterised in that** the emptying portion (2; 102; 202; 302) and the substance intake portion (1; 15; 201; 301) are arranged on the weighing balance (3), such that they are weighed by this weighing balance (3).

**25.** Device according to any one of claims 1 to 24, **characterised in that** the weighing balance (503) or a second weighing balance (503) is designed in order to receive a vessel (6) to be filled and to measure the weight of the vessel (6) and the substance (5; 50; 500) dosed into the vessel (6).

**26.** Method for dosage of substances with a device according to any one of claims 1 to 25, **characterised in that**

a) by emptying at least one substance compartment (11; 110; 111; 115; 211; 311; 330) of a substance intake portion (1; 15; 201; 301) containing substance (5; 50; 500), substance (5; 50; 500) is dosed into a vessel (6);
b) the quantity of dosed substance (5; 50; 500) is determined with a weighing balance (3; 503);
c) by control means it is calculated whether, and if need be, how much substance (5; 50; 500) is still to be dosed into the vessel (6), and according to result, it is proceeded further with step a) or the dosage is ended.

**27.** Method according to claim 26, **characterised in that** the substance intake portion (1; 15; 201; 301) comprises substance compartments (11; 110; 111; 115; 211; 311; 330) of varying size classes, and firstly, of the largest possible size class, the greatest number of substance compartments (11; 110; 111; 115; 211; 311; 330) are emptied in which it is still certain that the desired dosage quantity is not overshot, then, of the next smaller size class, the greatest number of substance compartments (11; 110; 111; 115; 211; 311; 330) in which it is still certain that the desired dosage quantity is not overshot are emptied, etc. until the desired dosage quantity with the desired precision is achieved.

**28.** Method according to claim 26 or 27, **characterised in that** the quantity of dosed substance (5; 50; 500) is determined after every emptying of a substance compartment (11; 110; 111; 115; 211; 311; 330).

**29.** Method according to claim 26 or 27, **characterised in that** the quantity of dosed substance (5; 50; 500) is determined only after the emptying of several substance compartments (11; 110; 111; 115; 211; 311; 330).

**30.** Method according to any one of claims 26 to 29, **characterised in that** the substance compartments (11; 110; 111; 115; 211; 311; 330) are filled before step a) by dipping in or insertion in substance (5; 50; 500) which is found in a supply container (4), and then afterwards taken out of the substance (5; 50; 500) again.

**31.** Method according to claim 30, **characterised in that** the weighing balance (3) measures the weight loaded on it before and after filling of the substance compartments (11; 110; 111; 115; 211; 311; 330), and the control means calculates from this, and from the known geometry of the individual substance compartments (11; 110; 111; 115; 211; 311; 330), the approximate quantity of substance (5; 50; 500) in each substance compartment (11; 110; 111; 115; 211; 311; 330).

**32.** Method according to claim 30 or 31, **characterised in that** after the first, preferably after every, emptying of a substance compartment (11; 110; 111; 115; 211; 311; 330) of a size class, the approximate quantity of substance (5; 50; 500) in a substance compartment (11; 110; 111; 115; 211; 311; 330) of this size class is newly estimated.

**33.** Method according to any of one claims 30 to 32, **characterised in that** after the filling of the substance compartments (11; 110; 111; 115; 211; 311; 330) firstly at least one substance compartment (11; 110; 111; 115; 211; 311; 330) of each size class is emptied and by generation of the weight difference before and after the emptying of each substance compartment (11; 110; 111; 115; 211; 311; 330), the approximate quantity of substance (5; 50; 500) in a substance compartment (11; 110; 111; 115; 211; 311; 330) of this size class is determined.

34. Method according to any one of claims 26 to 33, **characterised in that** dosing firstly takes place in an intermediate container and when the desired dosage quantity with the desired precision is achieved, the intermediate container is emptied into the vessel (6); whereas if the desired dosage quantity with regard to the desired precision is overshot, the intermediate container is emptied again and the dosage is begun again.

35. Method according to claim 34, **characterised in that** the actual dosage quantity in the intermediate container is determined by a second weighing balance, on which the intermediate container is fixed.

**Revendications**

1. Dispositif de dosage de substances, comprenant un dispositif de réception de substance (1 ; 15 ; 201 ; 301) qui comporte au moins un compartiment de substance (11 ; 110 ; 111 ; 115 ; 211 ; 311 ; 330) destiné à recevoir de la substance destinée à être dosée (5 ; 50 ; 500), un dispositif de vidage (2 ; 102 ; 202 ; 302) pour vider le au moins un compartiment de substance (11 ; 110 ; 111 ; 115 ; 211 ; 311 ; 330) et une balance (3 ; 503) pour déterminer la quantité de substance dosée (5 ; 50 ; 500), **caractérisé en ce que** le dispositif de réception de substance (1 ; 15 ; 201 ; 301) comprend une pluralité de compartiments de substances (11 ; 110 ; 111 ; 115 ; 211 ; 311 ; 330) qui peuvent être vidés séparément et **en ce que** le dispositif comprend en outre des moyens de commande qui commandent le vidage des compartiments de substance (11 ; 110 ; 111 ; 115 ; 211 ; 311 ; 330) en fonction de la quantité de substance dosée (5 ; 50 ; 500) déterminée au moyen de la balance (3 ; 503).

2. Dispositif selon la revendication 1, **caractérisé en ce que** le dispositif de réception de substance (1 ; 15 ; 201 ; 301) comprend des compartiments de substance (11 ; 110 ; 111 ; 115 ; 211 ; 311 ; 330) de classes de dimensions différentes permettant de recevoir des quantités différentes de substance destinée à être dosée (5 ; 50 ; 500).

3. Dispositif selon la revendication 2, **caractérisé en ce que** au moins quelques-unes des classes de dimensions sont échelonnées sur un facteur d'au moins 5, de préférence dans le rapport 1 : 2 : 5.

4. Dispositif selon l'une des revendications 1 à 3, **caractérisé en ce que** au moins quelques-uns des compartiments de substance (11 ; 110 ; 111 ; 115 ; 211 ; 311 ; 330) sont remplis à l'avance de substance destinée à être dosée (5 ; 50 ; 500) et de préférence fermés.

5. Dispositif selon l'une des revendications 1 à 4, **caractérisé en ce que** les compartiments de substance sont formés par des petits tubes (11 ; 110 ; 111 ; 211 ; 311 ; 330) disposés verticalement.

6. Dispositif selon la revendication 5, **caractérisé en ce que** les petits tubes (11 ; 110 ; 111 ; 211 ; 311 ; 330) présentent, par classes de dimensions, des diamètres intérieurs différents.

7. Dispositif selon la revendication 5 ou 6, **caractérisé en ce que** les diamètres intérieurs des petits tubes (11 ; 110 ; 111 ; 211 ; 311 ; 330) sont inférieurs à 5 mm, de préférence inférieurs à 1 mm, de manière plus préférée inférieurs à 0,5 mm, de manière encore plus préférée inférieurs à 0,1 mm.

8. Dispositif selon l'une des revendications 5 à 7, **caractérisé en ce que** au moins quelques-uns des petits tubes se rétrécissent du haut vers le bas.

9. Dispositif selon l'une des revendications 5 à 8, **caractérisé en ce que** au moins quelques-uns des petits tubes (110 ; 111) sont formés en pointe ou à arêtes vives en bas.

10. Dispositif selon l'une des revendications 5 à 9, **caractérisé en ce que** au moins quelques-uns des petits tubes (11 ; 110 ; 111 ; 211 ; 311 ; 330) sont remplis à l'avance de substance destinée à être dosée (5 ; 50 ; 500) et de préférence les deux extrémités des petits tubes (11 ; 110 ; 111 ; 211 ; 311 ; 330) sont fermées par un film (13, 14).

11. Dispositif selon l'une des revendications 1 à 10, **caractérisé en ce que** au moins quelques-uns des compartiments de substance (11 ; 110 ; 111 ; 115 ; 211 ; 311 ; 330) présentent une surface intérieure avec un indice de rugosité

moyenne arithmétique $R_a$ supérieur à 0,5 $\mu$m.

**12.** Dispositif selon l'une des revendications 1 à 11, **caractérisé en ce qu'**il présente différentes classes de compartiments de substance (11 ; 110 ; 111 ; 115 ; 211 ; 311 ; 330) avec des surfaces intérieures ayant des indices de rugosité arithmétique moyenne $R_a$ différents.

**13.** Dispositif selon l'une des revendications 1 à 12, **caractérisé en ce que** au moins quelques-uns des compartiments de substance (11 ; 110 ; 111 ; 115 ; 211 ; 311 ; 330) présentent sur leur surface intérieure des lamelles et/ou ardillons flexibles.

**14.** Dispositif selon l'une des revendications 1 à 13, **caractérisé en ce qu'**il présente différentes classes de compartiments de substance (11 ; 110 ; 111 ; 115 ; 211 ; 311 ; 330) avec des surfaces intérieures ayant une mouillabilité différente.

**15.** Dispositif selon l'une des revendications 1 à 14, **caractérisé en ce que** le dispositif de réception de substance (1 ; 15 ; 201 ; 301) est automatiquement séparable du dispositif de vidage (2 ; 102 ; 202 ; 302).

**16.** Dispositif selon l'une des revendications 1 à 15, **caractérisé en ce que** les compartiments de substance (11 ; 110 ; 111 ; 211 ; 311 ; 330) sont montés de manière individuelle dans le dispositif de réception de substance (1 ; 201 ; 301) et leur nombre est variable.

**17.** Dispositif selon l'une des revendications 1 à 16, **caractérisé en ce que** les compartiments de substance (11 ; 110 ; 111 ; 211 ; 311 ; 330) sont montés de façon réglable dans le dispositif de réception de substance (1 ; 201 ; 301) de manière individuelle entre une position de remplissage dans laquelle ils peuvent être remplis et une position inactive dans laquelle ils ne peuvent pas être remplis.

**18.** Dispositif selon l'une des revendications 1 à 17, **caractérisé en ce qu'**il comporte des moyens pour le réglage vertical du dispositif de réception de substance (1 ; 15 ; 201 ; 301).

**19.** Dispositif selon l'une des revendications 1 à 18, **caractérisé en ce que** le dispositif de vidage (2) comporte des moyens (22, 23) pour alimenter chacun des différents compartiments de substance (11) en gaz comprimé.

**20.** Dispositif selon l'une des revendications 1 à 19, **caractérisé en ce que** le dispositif de vidage (102) pour chaque compartiment de substance (11) présente un piston réglable (122).

**21.** Dispositif selon l'une des revendications 1 à 20, **caractérisé en ce que** le dispositif de vidage (202) présente des moyens pour modifier la géométrie de chaque compartiment de substance séparément (211 ; 330), lesquels moyens comprennent de préférence des moyens (222 ; 331 ; 332) pour produire une pression mécanique, une tension électrique ou une variation de température.

**22.** Dispositif selon l'une des revendications 1 à 21, **caractérisé en ce que** le dispositif de vidage (302) présente des moyens pour modifier les caractéristiques de surface de la surface intérieure de chaque compartiment de substance séparément (311), lesquels moyens comprennent de préférence des moyens (322 ; 331 ; 332) pour produire une tension électrique et/ou une variation de température.

**23.** Dispositif selon l'une des revendications 1 à 22, **caractérisé en ce que** le dispositif de vidage présente des moyens pour modifier les caractéristiques d'écoulement de la substance à doser (5 ; 50 ; 500) dans chaque compartiment de substance séparément, lesquels moyens comprennent de préférence des moyens pour produire une tension électrique ou une variation de température.

**24.** Dispositif selon l'une des revendications 1 à 23, **caractérisé en ce que** le dispositif de vidage (2 ; 102 ; 202 ; 302) et le dispositif de réception de substance (1 ; 15 ; 201 ; 301) sont disposés sur la balance (3) de manière à être pesés par celle-ci.

**25.** Dispositif selon l'une des revendications 1 à 24, **caractérisé en ce que** la balance (503) ou une deuxième balance (503) est conçue pour recevoir un récipient (6) à remplir et mesurer le poids du récipient (6) et de la substance dosée (5 ; 50 ; 500) dans le récipient (6).

**26.** Procédé pour doser des substances avec un dispositif selon l'une des revendications 1 à 25, **caractérisé en ce que**

a) par le vidage d'au moins un compartiment de substance (11 ; 110 ; 111 ; 115 ; 211 ; 311 ; 330) contenant une substance (5 ; 50 ; 500) d'un dispositif de réception de substance (1 ; 15 ; 201 ; 301) de la substance (5 ; 50; 500) est dosée dans un récipient (6) ;
b) avec une balance (3 ; 503) la quantité de substance (5 ; 50 ; 500) dosée est déterminée ;
c) par des moyens de commande, il est calculé si et, le cas échéant, combien de substance (5 ; 50 ; 500) reste encore à doser dans le récipient (6) et, suivant le résultat, on poursuit par l'étape a) ou on termine le dosage.

**27.** Procédé selon la revendication 26, **caractérisé en ce que** le dispositif de réception de substance (1 ; 15 ; 201 ; 301) comprend des compartiments de substance (11 ; 110 ; 111 ; 115 ; 211 ; 311 ; 330) de classes de dimensions différentes et le plus grand nombre de compartiments de substance (11 ; 110 ; 111 ; 115 ; 211 ; 311 ; 330) de la classe de dimensions la plus grande possible pour lequel nombre il est encore sûr que la quantité de dosage souhaitée n'est pas dépassée, est d'abord vidé, ensuite le plus grand nombre de compartiments de substance (11 ; 110 ; 111 ; 115 ; 211 ; 311 ; 330) de la classe de dimensions immédiatement plus petite pour lequel nombre il est encore sûr que la quantité de dosage souhaitée n'est pas dépassée, et ainsi de suite, jusqu'à ce que la quantité de dosage souhaitée soit atteinte avec la précision souhaitée.

**28.** Procédé selon la revendication 26 ou 27, **caractérisé en ce que** la quantité de substance dosée (5 ; 50 ; 500) est déterminée après chaque vidage d'un compartiment de substance (11 ; 110 ; 111 ; 115 ; 211 ; 311 ; 330).

**29.** Procédé selon la revendication 26 ou 27, **caractérisé en ce que** la quantité de substance dosée (5 ; 50 ; 500) est déterminée seulement après le vidage de plusieurs compartiments de substance (11 ; 110 ; 111 ; 115 ; 211 ; 311 ; 330).

**30.** Procédé selon l'une des revendications 26 à 29, **caractérisé en ce que** les compartiments de substance (11 ; 110 ; 111 ; 115 ; 211 ; 311 ; 330) sont remplis avant l'étape a) par immersion ou enfoncement dans la substance (5 ; 50 ; 500) qui se trouve dans un réservoir de stockage (4) et sont ensuite retirés de la substance (5 ; 50 ; 500).

**31.** Procédé selon la revendication 30, **caractérisé en ce que** la balance (3) mesure le poids la chargeant avant et après le remplissage des compartiments de substance (11 ; 110 ; 111 ; 115 ; 211 ; 311 ; 330) et les moyens de commande calculent à partir de cela et à partir de la géométrie connue des compartiments individuels de substance (11 ; 110 ; 111 ; 115 ; 211 ; 311 ; 330) la quantité approximative de matière (5 ; 50 ; 500) dans chaque compartiment de substance (11 ; 110 ; 111 ; 115 ; 211 ; 311 ; 330).

**32.** Procédé selon la revendication 30 ou 31, **caractérisé en ce que** après le premier, de préférence après chaque vidage d'un compartiment de substance (11 ; 110 ; 111 ; 115 ; 211 ; 311 ; 330) d'une classe de dimensions, la quantité approximative de substance (5 ; 50 ; 500) dans un compartiment de substance (11 ; 110 ; 111 ; 115 ; 211 ; 311 ; 330) de cette clase de dimensions est à nouveau estimée.

**33.** Procédé selon l'une des revendications 30 à 32, **caractérisé en ce que**, après le remplissage des compartiments de substance (11 ; 110 ; 111 ; 115 ; 211 ; 311 ; 330), d'abord au moins un compartiment de substance (11 ; 110 ; 111 ; 115 ; 211 ; 311 ; 330) de chaque classe de dimensions est vidé et la quantité approximative de substance (5 ; 50 ; 500) dans un compartiment de substance (11 ; 110 ; 111 ; 115 ; 211 ; 311 ; 330) de cette classe de dimensions est déterminée par formation de la différence de poids avant et après le vidage de chaque compartiment de substance (11 ; 110 ; 111 ; 115 ; 211 ; 311 ; 330).

**34.** Procédé selon l'une des revendications 26 à 33, **caractérisé en ce qu'**un dosage est effectué d'abord dans un récipient intermédiaire et, lorsque la quantité de dosage souhaitée est atteinte avec la précision souhaitée, le réservoir intermédiaire est vidé dans le récipient (6) tandis que, en cas de dépassement de la quantité dosée souhaitée, en tenant compte de la précision souhaitée, le réservoir intermédiaire est de nouveau vidé et on recommence le dosage.

**35.** Procédé selon la revendication 34, **caractérisé en ce que**, par une deuxième balance, à laquelle le réservoir intermédiaire est fixé, la quantité de dosage effective est déterminée dans le récipient intermédiaire.

Fig.1

2

12

11

1

x

y

Fig.2

122

2

1

12

11

# Fig.3

122
125
2
15
115

# Fig.4

2
12
4
5
11

Fig.5

2

4

50

12

11

Fig.6

500

2

12

11

Fig.7

110

Fig.8

111

Fig.9

14  12  11

5

13

Fig.10

23

22

N₂

21

2

12

11

1

5

Fig.11

122

121

12

102

11

1

5

Fig.12

221

202

222

212

211

201

5

Fig.13

222

221

212

211

Fig.14

331
330
4
5

321
332
312

Fig.15

321
312

Fig.16

331

321
332
312
6

Fig.17

331
301
50

302
332
321
322
312
311

Fig.18

321
322
312
311

Fig.19

3
7
8
2
12
1
11
6

Fig.20

8
2
1
6
503

Fig.21

3
7
8
2
1
6
503

**Fig.22**

Kalibration einer
Grössenklasse

nächste

Entleerung eines·
Substanzkompartiments

n erreicht?

nein

ja

m erreicht?

nein

ja

Berechnung der
Schwelle und der dazu
nötigen Zugaben

Grobdosierung

Wägen

Zielband
erreicht?

ja

Dosierung beendet

nein

Entleerung eines
Substanzkompartiments

K

G

F

Fig.23

EP 1 506 381 B1